# EUROPEAN PATENT SPECIFICATION

(11) **EP 3 986 268 B1**
(45) Date of publication and mention of the grant of the patent: **06.05.2026**
(21) Application number: 20826923.3
(22) Date of filing: 18.06.2020
(51) Int. Cl.: A61B 5/05, A61B 5/251, A61B 5/291, A61B 5/377, A61B 5/00, A61N 1/04, A61N 1/36

(54) **METHOD AND SYSTEM FOR MEASURING EEG SIGNALS**
VERFAHREN UND SYSTEM ZUR MESSUNG VON EEG-SIGNALEN
MÉTHODE ET SYSTÈME DE MESURE DE SIGNAUX D'EEG

(30) Priority: 18.06.2019 US 201962862689 P
(43) Date of publication of application: 27.04.2022
(73) Proprietor: Eeg-Sense Ltd., 5557202 Kiryat Ono (IL)
(72) Inventor: DEUTSCH, Israel, 4955336 Petach-Tikva (IL); LEVY, Yair, 1292200 Doar-Na Ramat HaGolan (IL)
(74) Representative: ip21 Ltd
(86) International application number: PCT/IL2020/050685
(87) International publication number: WO 2020/255142

(56) References cited:
- WO-A1-2012/140629
- WO-A1-2013/038285
- WO-A1-2018/109758
- CN-A- 108 742 605
- DE-A1- 102010 056 099
- US-A- 3 998 213
- US-A- 4 240 438
- US-A- 4 736 751
- US-A- 6 067 467
- US-A1- 2004 267 153
- US-A1- 2007 191 727
- US-A1- 2009 054 800
- US-A1- 2011 015 503
- US-A1- 2011 208 263
- US-A1- 2011 237 923
- US-A1- 2014 024 914
- US-A1- 2017 224 278
- US-A1- 2019 000 338
- US-A1- 2019 021 664

## Description

### RELATED APPLICATION

This application claims the benefit of priority of U.S. Provisional Patent Application No. 62/862,689 filed on June 18, 2019.

### FIELD AND BACKGROUND OF THE INVENTION

The present invention, in some embodiments thereof, relates to electroencephalography (EEG) and, more particularly, but not exclusively, to a method and system for measuring EEG signals.

The brain is a complex structure of nerve cells that produce signals called excitatory post synaptic potentials (EPSP). These potentials summate in the cortex and extend through the coverings of the brain to the scalp, where they can be measured using appropriate electrodes. Rhythmical measured activity represents postsynaptic cortical neuronal potentials which are synchronized by the complex interaction of large populations of cortical cells. EEG, a noninvasive recording technique, is one of the commonly used systems for monitoring brain activity. EEG data is simultaneously collected from a multitude of channels at a high temporal resolution, yielding high dimensional data matrices for the representation of brain activity. In addition to its unsurpassed temporal resolution, EEG is non-invasive, wearable, and more affordable than other neuroimaging techniques.

In some techniques, EEG signals are acquired by headsets.

One very common example of an EEG headset used in medical applications is a standard EEG cap, which includes a cap made of a rubber or rubber-like material that is put on the subject's head like a swimming cap. The cap has surface electrodes positioned throughout the cap in a manner such that they come in contact with surface of the subject's head. Common contemporary EEG headsets require extensive preparation along with professional lengthy setup and positioning. In order to improve conductivity, gel or saline is typically employed. In some cases, uncomfortable pressure is applied to the electrodes to enhance the electrical coupling. Also known, is the use of dry electrodes, that allow fast and easy setup. Once an EEG headset is worn, a trial-and-error procedure is employed to ensure that the headset is properly aligned with the desired locations on the scalp and properly attached to the scalp to achieve good conductivity.

### SUMMARY OF THE INVENTION

The invention is defined by the appended claims. According to an aspect of some embodiments of the present disclosure there is provided a system for measuring electroencephalography (EEG) signals. The system comprises a wearable body adapted to fit over a scalp, a plurality of electrodes mounted on the wearable body at a density of at least 2 electrodes per 3 cm², and a signal processor configured for detecting Evoked Related Potential (ERP) signals from the electrodes and determining a physiological location of each electrode or each group of electrodes based on the ERP signals.

According to some embodiments of the disclosure the system comprises an input for receiving from a stimulation system signals describing stimulation of a subject wearing the wearable body, wherein the signal processor configured for determining the location based in part on the signals from the stimulation system.

According to some embodiments of the disclosure the stimulation system is configured to apply electrical stimulation by at least one of the electrodes.

According to an aspect of some embodiments of the present disclosure there is provided a system for measuring EEG signals. The system comprises: a wearable body adapted to fit over a scalp; a plurality of electrodes mounted on the wearable body; a plurality of stretch sensors for generating stretch data describing a stretching of the wearable body; and a signal processor configured for receiving the stretch data and constructing from the stretch data a three-dimensional map describing physiological locations of the electrodes over the wearable body, once stretched.

According to some embodiments of the disclosure the system comprises a plurality of physically separate sensing systems, each comprising several of the plurality of electrodes.

According to an aspect of some embodiments of the present disclosure there is provided a system for measuring EEG signals. The system comprises: a wearable body adapted to fit over a scalp; a plurality of electrodes mounted on the wearable body; a plurality of controllable actuators for applying force to the electrodes; a controller configured for individually controlling each actuator or group of actuators to apply force to at least one electrode; and a signal processor configured for receiving and processing signals from the electrodes and transmitting control signals to the controller based on the processing.

According to some embodiments of the disclosure the signal processor is configured for determining at least one of: an electrode-tissue impedance, a signal-to-noise ratio, artifacts percentage, and a signal quality, and to control the force based on the determination.

According to some embodiments of the disclosure at least one of the electrodes is flexible and configured to experience a strain once pressed by the force against the scalp.

According to some embodiments of the disclosure the actuator is configured to apply the force while establishing rotary motion to the electrodes.

According to some embodiments of the disclosure at least one of the controllable actuators comprises an inflatable balloon, applying the force upon inflation thereof.

According to some embodiments of the disclosure the force is periodic and is applied to vibrate the electrodes or generate a hammering effect.

According to some embodiments of the disclosure the system comprises a plurality of physically separate sensing systems, each comprising several of the plurality of electrodes.

According to some embodiments of the disclosure at least one of the sensing systems comprises a circuit board and a plurality of flexible legs, each having a non-conductive section and a conductive section having a tip in electrical communication with the circuit board, wherein each conductive section is one of the plurality of electrodes.

According to some embodiments of the disclosure the circuit board and the plurality of flexible legs are detachable from each other.

According to some embodiments of the disclosure the wearable body comprises an inner shell supporting the circuit board and the plurality of flexible legs, and an outer shell supporting the plurality of controllable actuators.

According to some embodiments of the disclosure at least one of the flexible legs has a helical shape.

According to some embodiments of the disclosure a conductive section of at least one of the plurality of legs is polymeric.

According to some embodiments of the disclosure a conductive section of at least one of the plurality of legs comprises a bundle of conductive bristles.

According to some embodiments of the disclosure the system comprises a controllable vibrating member configured for vibrating the legs.

According to some embodiments of the disclosure at least one of the legs comprises a hydrophobic zone at an upper part of the leg and a hydrophilic zone at a lower part of the leg.

According to some embodiments of the disclosure the plurality of flexible legs is arranged on a base of a sensing system body, wherein the sensing system(s) comprises a shaft and a housing mounted on the shaft and being configured to receive the sensing system body, and wherein the housing comprises a rigid wall for holding the sensing system body and a flexible membrane connecting the rigid wall with the shaft in a manner that allows the housing to assume a plurality of different orientations with respect to the shaft.

According to some embodiments of the disclosure the system comprises a controller for controlling a connection state of each individual electrode.

According to some embodiments of the disclosure the system comprises a wired bus interface for establishing communication between the controller and the electrodes.

According to some embodiments of the disclosure the controller is configured for electrically grouping the electrodes into at least one group.

According to some embodiments of the disclosure the group(s) forms a predetermined morphology over a surface of the scalp.

According to some embodiments of the disclosure the group(s) is selected in a closed loop control based on at least one of: an electrode-tissue impedance, a signal-to-noise ratio, artifacts percentage and a signal quality, calculated by the signal processor for signals received by the electrodes.

According to an aspect of some embodiments of the present disclosure there is provided a method of measuring EEG signals. The method comprises operating the system as disclosed above, while the wearable body is placed on a scalp of a subject, to receive EEG signals sensed by the plurality of electrodes, thereby measuring the EEG signals.

According to an aspect of some embodiments of the present disclosure there is provided a system for sensing electroencephalography signals. The system comprises a circuit board and a plurality of flexible legs, each having a non-conductive section and a conductive section having a tip in electrical communication with the circuit board, wherein at least one of the flexible legs has a helical shape.

According to an aspect of some embodiments of the present disclosure there is provided a system for sensing electroencephalography signals. The system comprises a circuit board and a plurality of flexible legs, each having a non-conductive section and a conductive section having a tip in electrical communication with the circuit board, wherein a conductive section of at least one of the plurality of legs comprises a bundle of conductive bristles.

According to an aspect of some embodiments of the present disclosure there is provided a system for sensing electroencephalography signals. The system comprises a circuit board and a plurality of flexible legs, each having a non-conductive section and a conductive section having a tip in electrical communication with the circuit board, wherein at least one of the legs comprises a hydrophobic zone at an upper part of the leg and a hydrophilic zone at a lower part of the leg.

According to some embodiments of the disclosure the leg(s) comprises an intermediate zone between the hydrophobic zone and the hydrophilic zone, the intermediate zone being less hydrophobic than the hydrophobic zone, and less hydrophilic than the hydrophilic zone.

According to an aspect of some embodiments of the present disclosure there is provided a method of measuring EEG signals. The method comprises: detecting Evoked Response Potential (ERP) signals from a plurality of electrodes placed on a surface scalp of a subject at a density of at least 2 electrodes per 3 cm²; and processing the ERP to determine a physiological location of each electrode or each group of electrodes on the surface based on the ERP signals.

According to some embodiments of the disclosure the method comprises receiving from a stimulation system signals input describing stimulation of the subject, wherein the determination of the location is based in part on the signals from the stimulation system.

According to an aspect of some embodiments of the present disclosure there is provided a method of measuring EEG signals. The method comprises: receiving signals from a plurality of electrodes placed on a surface scalp of a subject; and processing the signals and controlling a plurality of controllable actuators to apply force to the electrodes, based on the processing.

According to some embodiments of the disclosure the method comprises determining at least one quantity selected from the group consisting of: an electrode-tissue impedance, a signal-to-noise ratio, an artifacts percentage, and a signal quality, and increasing the force responsively to a value of the at least one quantity.

According to some embodiments of the disclosure the force is applied inwardly.

According to some embodiments of the disclosure at least one of the controllable actuators comprises an inflatable balloon, wherein the controlling comprising inflating the balloon to apply the force.

According to some embodiments of the disclosure the method comprises determining a pressure in the balloon and at least one quantity selected from the group consisting of: an electrode-tissue impedance, a signal-to-noise ratio, an artifacts percentage, and a signal quality, and, varying the pressure responsively to a value of the at least one quantity.

According to some embodiments of the disclosure the controlling comprises operating the actuators to apply the force periodically so as to vibrate the electrodes or generate a hammering effect.

According to some embodiments of the disclosure the method comprises determining at least one quantity selected from the group consisting of: an electrode-tissue impedance, a signal-to-noise ratio, an artifacts percentage, and a signal quality, and operating the actuators to vibrate the electrodes or generate the hammering effect responsively to a value of the at least one quantity.

According to some embodiments of the disclosure the method comprises varying a connection state of at least one of the electrodes.

According to some embodiments of the disclosure the method comprises electrically grouping the electrodes into at least one group.

According to some embodiments of the disclosure the method comprises selecting the group(s) in accordance with a predetermined morphology over a surface of the scalp.

According to some embodiments of the disclosure the method comprises determining at least one quantity selected from the group consisting of: an electrode-tissue impedance, a signal-to-noise ratio, an artifacts percentage, and a signal quality, and selecting the group(s) in a closed loop control based on a value of the quantity(ies).

According to an aspect of some embodiments of the present disclosure there is provided a jig system for collectively assembling a plurality of sensing systems, each sensing system being configured for sensing EEG signals. The jig system comprises a scaffold having an outer surface and an inner surface, the outer surface being designed and constructed to fittedly receive a wearable body mounted with a plurality of housings; a plurality of recesses formed in the outer surface, each having a base and a through hole formed between the base and the inner surface of the scaffold, each recesses being size-wise and shape-wise compatible with a disposable electrode assembly of one of the sensing systems; and a plurality of jig shafts, each introduced into one of the recesses from a side of the inner surface via a respective through holes.

According to an aspect of some embodiments of the present disclosure there is provided a kit. The kit comprises the jig system as delineated above and optionally and preferably as further detailed below; and a system for measuring EEG signals, wherein the system comprises a wearable body adapted to fit over a scalp, and a plurality of housings mounted on the wearable body, each housing having therein a circuit board of one of the sensing systems, and being configured to receive an electrode assembly of one of the sensing systems.

According to some embodiments of the disclosure the kit comprises a plurality of disposable electrode assemblies each being size-wise and shape-wise compatible with one of the recesses.

According to an aspect of some embodiments of the present disclosure there is provided a method of assembling a system for measuring EEG signals. The method comprises placing a plurality of disposable electrode assemblies of a respective plurality of sensing systems in a respective plurality of recesses of a jig system such as, but not limited to, the jig system delineated above and optionally and preferably as further detailed below; mounting on the scaffold a wearable body having thereon a plurality of housings each being configured to receive one of the electrode assemblies, and having therein a circuit board of one of the sensing systems, so as to align the housings with the recesses; and activating the jig shafts to release the electrode assemblies out of recesses and to connect the electrode assemblies to the housings.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the disclosure, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the disclosure can involve performing or completing selected tasks manually, automatically, or a combination thereof. Moreover, according to actual instrumentation and equipment of embodiments of the method and/or system of the disclosure, several selected tasks could be implemented by hardware, by software or by firmware or by a combination thereof using an operating system.

For example, hardware for performing selected tasks according to embodiments of the disclosure could be implemented as a chip or a circuit. As software, selected tasks according to embodiments of the disclosure could be implemented as a plurality of software instructions being executed by a computer using any suitable operating system. In an exemplary embodiment of the disclosure, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

### BRIEF DESCRIPTION OF THE SEVERAL VIEWS OF THE DRAWINGS

Some embodiments of the disclosure are herein described, by way of example only, with reference to the accompanying drawings. With specific reference now to the drawings in detail, it is stressed that the particulars shown are by way of example and for purposes of illustrative discussion of embodiments of the disclosure.

In this regard, the description taken with the drawings makes apparent to those skilled in the art how embodiments of the disclosure may be practiced.

In the drawings:
FIGs. 1A and 1B are schematic illustrations of a system for measuring EEG signals, according to some embodiments of the present invention;
FIG. 1C is a schematic illustration showing a mounting configuration of electrodes on a wearable body according to some embodiments of the present invention;
FIGs. 1D-F are schematic illustrations showing representative examples of shapes suitable for the electrodes shown in FIG. 1C, according to some embodiments of the present invention;
FIGs. 2A-E are schematic illustrations of a wearable body which comprises a plurality of stretch sensors, according to some embodiments of the present invention;
FIGs. 3A-F are schematic illustrations of a sensing system that comprises several electrodes, in embodiments of the invention in which metallic wires are employed;
FIGs. 4A-F are schematic illustrations of a sensing system that comprises several electrodes, in embodiments of the invention in which a bundle of conductive bristles is employed;
FIGs. 5A-F are schematic illustrations of a sensing system that comprises several electrodes, in embodiments of the invention in which a bundle of a conductive polymer is employed;
FIG. 6 is a schematic illustration of a helical sensing leg, according to some embodiments of the present invention;
FIG. 7 is a schematic illustration of a sensing leg in embodiments of the invention in which the leg comprises a hydrophobic zone and a hydrophilic zone;
FIGs. 8A-H are schematic illustrations of a system a system for measuring EEG signals in embodiments of the invention in which the system comprises controllable actuators for applying force to the electrodes;
FIGs. 9A and 9B are schematic illustrations of the system in embodiments of the invention in which the actuator comprises a balloon, a shaft, and a stator, and the wearable body comprises an outer shell and an inner shell;
FIG. 9C is a schematic illustration of a sheet member and a plurality of bodies of sensing systems embedded therein, according to some embodiments of the present invention;
FIGs. 10A and 10B are schematic illustrations describing a bus interface according to some embodiments of the present invention;
FIG. 11 is a flowchart diagram describing a close loop procedure for operating an actuator to apply a compression force on an electrode, according to some embodiments of the present invention;
FIG. 12 is a flowchart diagram describing a close loop procedure for operating an actuator to vibrate the electrodes, according to some embodiments of the present invention;
FIG. 13 is a flowchart diagram describing a close loop procedure for operating an actuator to provide a hammering effect, according to some embodiments of the present invention;
FIGs. 14A-F are schematic illustrations showing additional configurations of a sensing leg according to some embodiments of the present invention;
FIG. 15 is a flowchart diagram describing a combined procedure for operating an actuator, according to some embodiments of the present invention; and
FIGs. 16A-D which are schematic illustrations of a jig system according to some embodiments of the present invention.

### DESCRIPTION OF SPECIFIC EMBODIMENTS OF THE INVENTION

The present invention, in some embodiments thereof, relates to EEG and, more particularly, but not exclusively, to a method and system for measuring EEG signals and is as specified in the claims.

Before explaining at least one embodiment of the invention in detail, it is to be understood that the invention is not necessarily limited in its application to the details of construction and the arrangement of the components and/or methods set forth in the following description and/or illustrated in the drawings and/or the Examples.

The inventors realized that there are several technological challenges in a measurement of EEG signals. EEG signals can be collected using wet electrodes or dry electrodes. It was realized by the inventors that using wet electrodes requires time consuming preparations, skilled experienced operators and complicates the process, because a conductive gel should be applied on the scalp, and because subject's preparation oftentimes requires hair shaving and removal of dead skin from the scalp. The gel may also cause discomfort to the subject, and may also result in skin irritation. Additionally, since the gel is drying over time, it needs to be reapplied regularly (typically every hour). Further, the amount of applied gel needs to be considered with great care, since too much gel may cause short-circuits between electrodes, and to less gel may result in poor conductivity.

It was realized by the inventors that the use of dry electrodes, is not without certain operative limitations that would best be avoided. These include the need to manually place the electrodes one by one and to apply pressure on the electrodes so as to ensure penetration through the hair and good contact. The applied pressure may also cause discomfort since the subject may suffer from constant tension on the scalp. Use of dry electrode may also cause discomfort due to entanglement between the electrodes and the hair.

Furthermore, known EEG systems include a wired cable per electrode. This limits the ability of the subject to move, and may also reduce the signal quality since unavoidable movements of the subject may result in signal disruptions (artifacts).

Additionally, in traditional EEG systems, there is a requirement for accurate placement of each electrode on a specific location over the scalp (e.g., according to the International 10-20 EEG scheme). The Inventors realized that deviations from the specific locations can reduce the accuracy of the diagnostics.

In a search for a solution to the above problems the Inventors devised a technique for sensing and measuring EEG signals. The technique of the present embodiments can be used to investigate and/or diagnose various conditions and disorders, including, without limitation, seizures, traumatic brain injury (TBI), hemorrhages, brain tumors, encephalopathy, cognitive decline, sleep disorders, ischemic pathologies, *e.g.*, stroke, dementia and coma level of brain activity. The technique of the present embodiments can be used to evaluate several types of brain disorders. For example, when epilepsy is present, seizure activity can appear as rapid spiking waves on the EEG, and when the brain includes lesions, which can result from tumors or stroke, unusually slow EEG waves can be detected, depending on the size and the location of the lesion. The technique of the present embodiments can be used in biofeedback applications for various purposes, including, without limitation, improving the quality of life for subjects suffering from motor disorders or motor dysfunction.

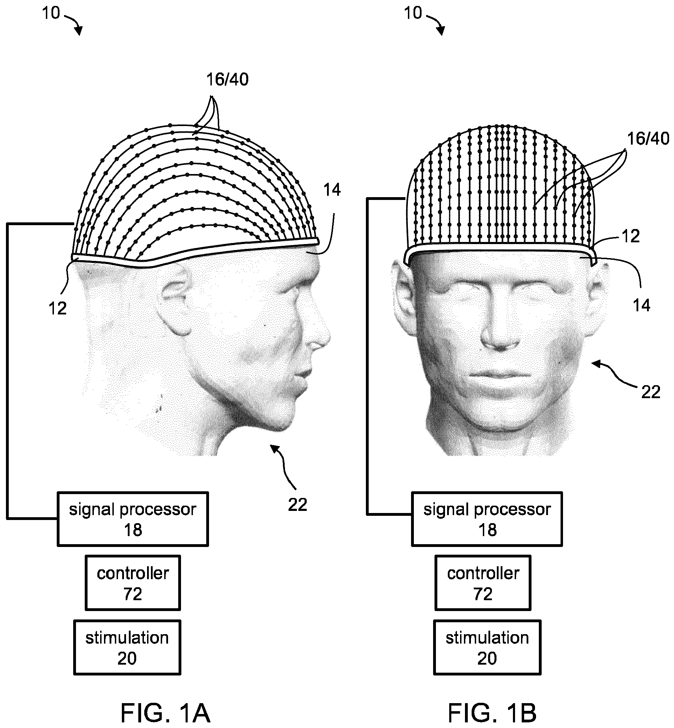

Referring now to the drawings, FIGs. 1A and 1B are schematic illustrations of a system **10** for measuring EEG signals, according to some embodiments of the present disclosure. In various exemplary embodiments of the disclosure preferably, but not necessarily, system **10** is a dry EEG system, in the sense that the system can measure EEG signals by establishing contact with a scalp **14** of a subject **22** in dry environment, and in particular without applying gel between the sensing elements and the skin.

System **10** preferably comprises a wearable body **12** adapted to fit over scalp **14.** Wearable body **12** can be rigid, flexible, or elastic, and can be made of any material that can be worn over the scalp, such as, but not limited to, a synthetic fabric, molded plastic, fiberglass, reinforced fiberglass (*e.g*., reinforced with Kevlar or carbon fibers).

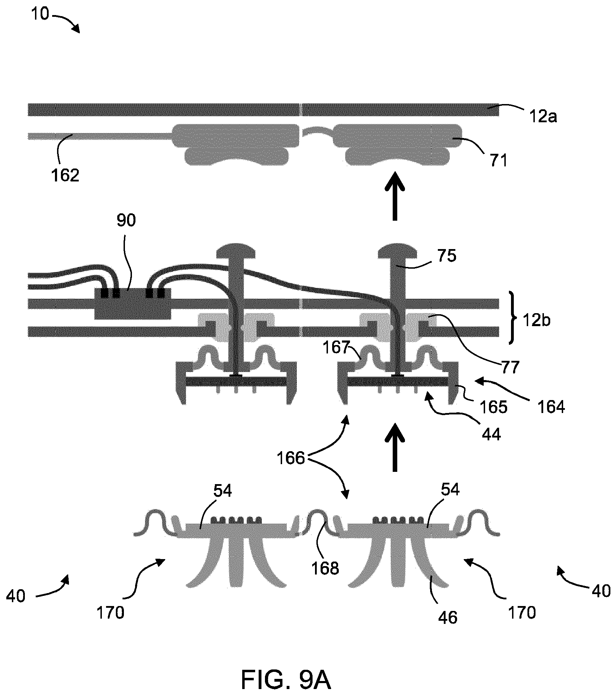

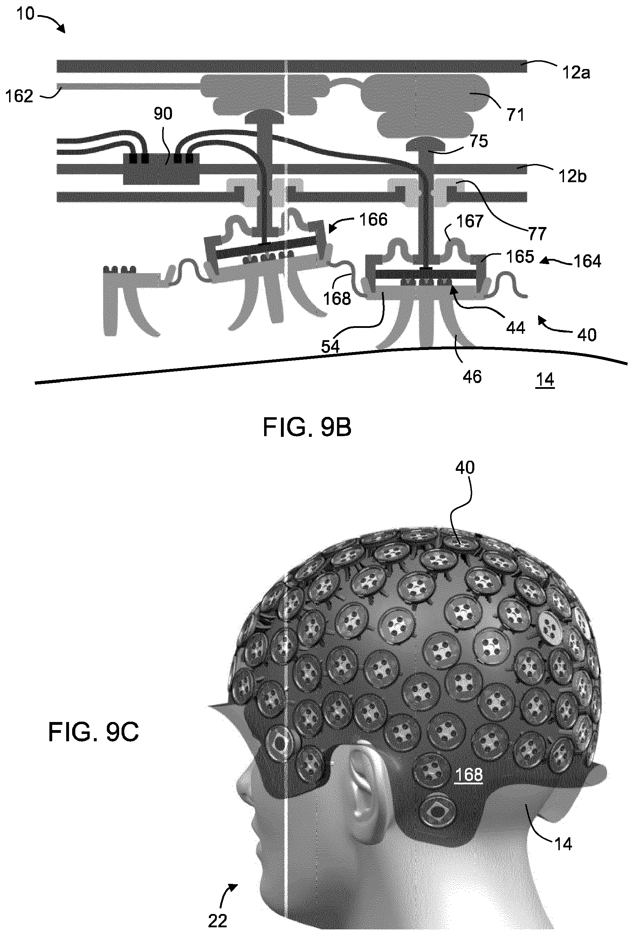

In some embodiments of the present invention wearable body **12** comprises an outer shell and an inner shell (not shown in FIGs. 1A and 1B, see FIGs. 9A and 9B described below). In some embodiments of the present disclosure at least a portion of wearable body **12** is elastic. System **10** further comprises a plurality of electrodes **16** mounted on wearable body **12.** Preferably, but not necessarily, system **10** comprises a plurality of sensing systems **40,** each comprising several electrodes, so that electrodes **16** are physically grouped into a plurality of separate sensing systems **40.** A more detailed description of sensing systems suitable for the present embodiments is provided hereinunder.

The number of electrodes **16** that are distributed over wearable body **12** is preferably at least 16 or at least 32 or at least 64 or at least 128 or at least 256 or at least 512 or more. Preferably, electrodes **16** are distributed over wearable body **12** at a density of at least 2 electrodes per 3 cm² or at least 4 electrodes per 3 cm² or at least 8 electrodes per 3 cm², or at least 2 electrodes per 2 cm² or at least 4 electrodes per 2 cm² or at least 8 electrodes per 2 cm², or at least 2 electrodes per cm² or at least 4 electrodes per cm² or at least 8 electrodes per cm². For at least 50% or at least 60% or at least 80% of the electrodes, the distance between adjacent electrodes is preferably less than 3 cm or less than 2.5 cm or less than 2 cm or less than 1.5 cm or less than 1 cm.

Use of large number of electrodes, according to preferred embodiments of the present disclosure allows system **10** to perform high-density EEG (hdEEG) which can provide high spatial sampling density as well as large head coverage.

hdEEG allows the recording of spontaneous or evoked brain activity with improved spatial resolution. hdEEG can provide spatial resolution (number of channels) that is sufficiently high for the investigation of various conditions such as, but not limited to, epilepsy, cognitive processes, brain Injury and neuropsychiatric disorders, as well as for detecting deep brain activities. hdEEG can be used, for example, for accurate localization of Epileptic foci, and/or neural network investigation, *e.g*., assessment of functional connectivity, and utilization in brain computer interface (BCI) systems.

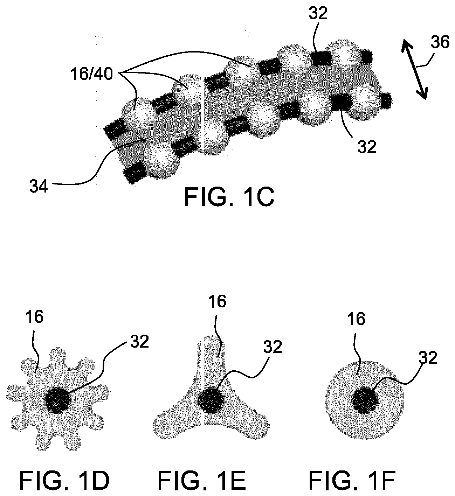

A mounting configuration of electrodes **16** on wearable body **12** according to some embodiments of the present invention is illustrated in FIG. 1C. In these embodiments, electrodes **16** are mounted on flexible strings **32** that are attached gridwise to wearable body **12** (not shown in FIG. 1C, see, *e.g.,* FIGs. 1A and 1B). In some embodiments, electrodes **16** are distributed uniformly along strings **32.** In embodiments in which system **10** comprises a plurality of sensing systems **40,** each comprising several electrodes, the sensing systems **40** are preferably, but not necessarily, distributed uniformly along strings **32.**

In some embodiments of the present disclosure, an elastic band **34** is connected to strings **32.** In the illustration shown in FIG. 1C, band **34** connects two strings **32** so that when band **34** is stretched (for example, while wearable body **12** is worn on scalp **14** strings **32** are displaced from each other along a direction generally orthogonal to strings **32,** as generally shown by doublehead arrow **36.** Electrodes **16** can have any shape. Representative examples of shapes suitable for electrodes **16** on string **32** are illustrated in FIGs. 1D-F, showing a chain wheel shape (FIG. 1D), a tripod shape (FIG. 1E), and a disc or ball shape (FIG. 1F). Other shapes are also contemplated in some embodiments of the present disclosure.

Optionally, system **10** comprises a controller **72** having a circuit configured for controlling a connection state of each individual electrode. For example, controller **72** can group the electrodes electrically into two or more groups. The grouping is electrical in the sense that the signals sensed by all electrodes in a group are coherently combined so that the combined signal represents a sensing event from an area over the scalp **14** that is occupied by all the electrodes in the group. The groups can be defined based on one or more criteria. For example, in some embodiments of the present disclosure the groups are selected based on a predetermined morphology over a surface of the scalp, and in some embodiments of the present disclosure the groups are selected in a closed loop control based on an impedance or a signal-to-noise ratio of signals received by the electrodes, and in some embodiments of the present disclosure the groups are selected based on an independent signal analysis. Grouping operations according to some embodiments of the present disclosure are provided in greater detail hereinunder.

System **10** optionally and preferably also comprises a signal processor **18** having one or more circuits that receive and process EEG signals sensed by electrodes **16,** and that optionally and preferably transmit control signals to controller **72.** Processor **18** can be configured for executing any of the processing operations described herein. Typically, processor **18** comprises an Analog-to-Digital (A2D) circuit that digitizes the signals sensed by electrodes **16,** and a digital signal processing (DSP) circuit that receives the digitized signals from the A2D circuit and applies digital signal processing operations to the digitized signals. Preferably, but not necessarily, processor **18** comprises a dedicated circuit, for example, an application-specific integrated circuit (ASIC) configured for executing these operations. Also contemplated is the use of a field-programmable gate array (FPGA) for performing at least a few of the image processing operations.

Communication between electrodes **16** and processor **18** is optionally and preferably by means of a wired bus interface, as further detailed hereinbelow. Processor **18** can be provided as a separate unit or be mounted on wearable body **12.** Also contemplated are embodiments in which processor **18** comprises a first circuit that is mounted on wearable body **12** and a second circuit that is not mounted on wearable body **12,** wherein one or more processing operations are performed by the circuit that is mounted on wearable body **12** and the other operations are performed by the circuit that is not mounted on wearable body **12.** For example, the A2D circuit can be mounted on wearable body **12,** and the DSP circuit can be distant to wearable body **12.**

In some embodiments of the present disclosure processor **18** is configured for detecting Evoked Response Potential (ERP) signals from electrodes **16** and determining a physiological location of each electrode or each group of electrodes based on the ERP signals. In these embodiments, the density of the electrodes is preferably large (*e.g.,* at least 2 electrodes per 3 cm² or at least 4 electrodes per 3 cm² or at least 8 electrodes per 3 cm², or at least 2 electrodes per 2 cm² or at least 4 electrodes per 2 cm² or at least 8 electrodes per 2 cm², or at least 2 electrodes per cm² or at least 4 electrodes per cm² or at least 8 electrodes per cm²), so as to improve the resolution of the location determination.

An ERP is the body's psychophysiological response to a given stimulus. Since individual neurons have relatively little electrical activity associated with them, certainly not enough to be detected on the scalp, ERPs are recorded when neurons act synchronously, and the electric fields generated by each particular neuron are oriented in such a way that the effects on the scalp cumulate. Activity of neurons organized in a layered open field manner (neurons with dendrites and axons oriented in the same fashion) are typically picked up as an ERP. Stimuli that cause ERPs can either be external, or internal.

The detection of ERP signals is optionally and preferably performed by processor **18** based on signals that describe stimulation of subject **22** wearing a wearable body **12.** These signals are typically received from a stimulation system **20.** Processor **18** preferably synchronizes between the signals from stimulation system **20** and the EEG signals from electrodes **16** to identify the ERP signals among the EEG signals. Stimulation system **20** can apply any type of stimulation, including, without limitation, somatosensory evoked potential (SSEP), brainstem evoked response audiometry, visual stimulation, tactile stimulation, olfactory stimulation and motor stimulation.

For any identified ERP signal, processor **18** can determine at which of electrodes **16** the ERP signal is sensed and determine the location of the electrode(s) based on the type of stimulus that evoked the ERP signal. In various exemplary embodiments of the disclosure several stimuli are applied to subject **22,** and the identification of the respective ERP signal and the location of the electrode(s) that sense the respective ERP signal is repeated for each of at least a few of the applied stimuli. Preferably, a morphological map of the electrodes over the anatomical areas of the cortex is generated based on the determined locations. Thus, unlike conventional techniques, such as the 10-20 EEG scheme, in which each electrode must be placed at a specific location, the present embodiments advantageously generate a morphological map that automatically associates each electrode or group of electrodes to a specific anatomical area of the cortex.

For example, using SSEP sensory stimulation on the motor nerve system the maximal recorded P20 can localize the sensory cortex (post central gyrus). By localizing several anatomical locations and knowing the dispositions between the electrodes and sensing points, all electrodes can be mapped and tailored to individual's anatomy. The other electrodes can be mapped based on their physical distance from mapped electrodes. Another example is a stimulus in which visual patterns of specific images, such as, but not limited to, checkboards, or flicker lights, are displayed to induce visual evoked potentials can be induced. An additional example is a sound stimulus in which a specific sound (*e.g.,* at a specific frequency, duration and/or amplitude) is presented to subject **22.** Also contemplated, are electronic stimulations, such as, but not limited to, applying external stimulation to an organ, *e.g.*, using a wrist stimulation device for applying an electric pulse to from the wrist. Such a stimulus induces an ERP signal in the sensory part of the brain and can thus be used to determine the location of the electrode(s) picking this signal. Further contemplated, are embodiments in which an electric pulse is transmitted to the scalp by one or more of the EEG electrodes and identifying the ERP signals indicted by this stimulation. In these embodiments, stimulation system **20** is optionally and preferably in direct communication with electrodes **16** and is configured to transmit electronic signals to the scalp via the respective electrode or electrodes.

Additional stimulation types and the corresponding cortex area at which an ERP signals is induced, are described in the Examples section that follows.

It is appreciated that multiplicity of stimulations can be applied simultaneously or sequentially. Preferably the applied stimulations are synchronous thereamongst.

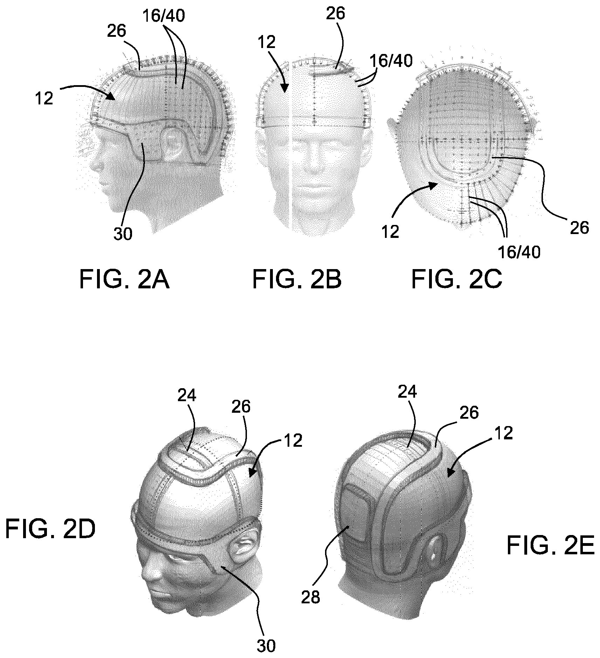

In some embodiments of the present disclosure wearable body **12** comprises a plurality of stretch sensors. These embodiments are illustrated in FIGs. 2A-E. Shown in FIGs. 2A-E are stretch sensors **24** mounted on wearable body **12.** In some embodiments of the present disclosure stretch sensors **24** are woven into strings **32** (not shown, see FIG. 1C) on which electrodes **16** are mounted. Embodiments in which the stretch sensors are employed without strings **32** are also contemplated according to some embodiments of the present disclosure.

Wearable body **12** is preferably also provided with rigid arcs **26** for providing mechanical reinforcement to body **12** and may optionally include a hardware component **28** that may include one or more a bus architecture and a controller, as further detailed hereinbelow, and/or a power source **30.** Additional configurations for wearable body **12** are described hereinunder.

Stretch sensors **24** convert analog motion or tension to stretch data describing a stretching of wearable body **12.** Signal processor **18** (not shown, see FIGs. 1A and 1B) optionally and preferably receives the stretch data from sensors **24** and constructs from the stretch data a three-dimensional map describing the locations of electrodes **16** over the scalp, once wearable body **12** is stretched. Sensors suitable for use as stretch sensors **24** are disclosed in U.S. Published Application No. 20160238368, and are commercially available from StretchSense^{™}, Auckland, New Zealand.

Typically, processor **18** constructs the three-dimensional map also using one or more reference points for which the locations are known. In these embodiments the wearable body is worn and stretched such that one or more reference points that are marked on the wearable body are aligned with specific anatomical points (*e.g.,* ears, eyebrows, forehead midline, *etc*.). Preferably, signal processor is programed in advance with information pertaining to the length in rest state of the elastic band **34,** or the inter-string distance when elastic band **34,** is not stretched. Using this information and the stretch data from sensors **24,** processor **18** calculates the interelectrode distances, and using the calculated distances and the location of the reference points, processor **18** constructs the three-dimensional map.

The three-dimensional map allows system **10** to create a cross-correlation between the brain activity axis system and the physical positioning of electrodes **16** and localize a specific area of interest at both the brain simulation and the scalp on the anatomical level with high precision.

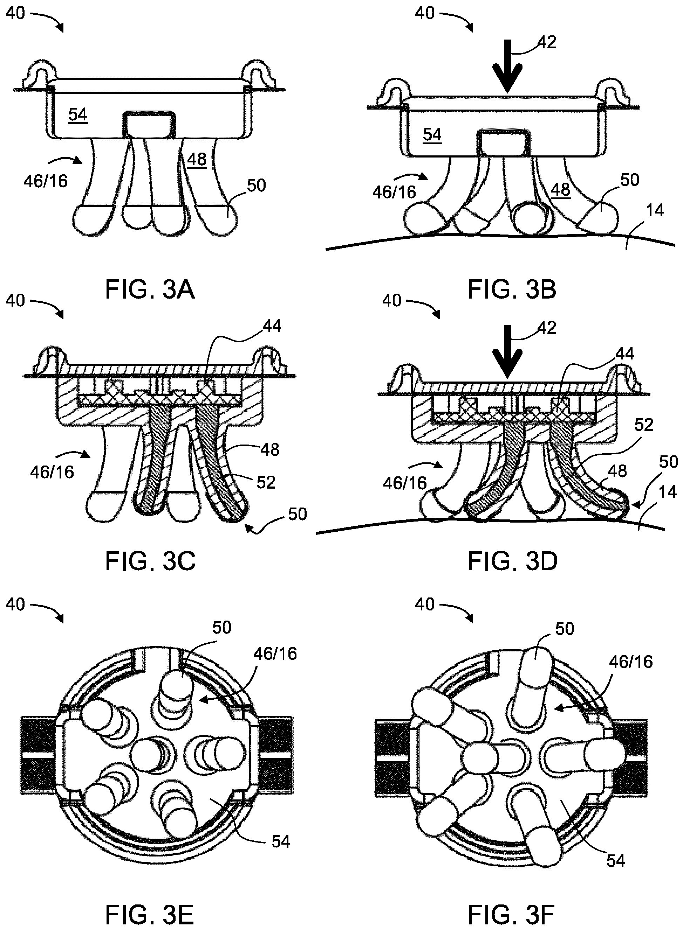

Reference is now made to FIGs. 3A-F, 4A-F, and 5A-F, which are schematic illustrations of a sensing system **40** that comprises several electrodes **16,** according to various exemplary embodiments of the present invention. Shown are front views (FIGs. 3A-B, 4A-B, and 5A-B), cross-sectional views (FIGs. 3C-D, 4C-D, and 5C-D), and bottom view (FIGs. 3E-F, 4E-F, and 5E-F) of system **40** in a relaxed state (FIGs. 3A, 3C, 3E, 4A, 4C, 4E, 5A, 5C, and 5E) and in a compressed state (FIGs. 3B, 3D, 3F, 4B, 4D, 4F, 5B, 5D, and 5F), once a compression force **42** is inwardly applied to sensing system **40** in the direction of scalp **14.** A plurality of sensing systems like system **40** can be distributed over wearable body **12** of system **10,** for example, along strings **32,** as further detailed hereinabove.

Sensing system **40** typically comprises a circuit board **44,** *e.g.,* a printed circuit board, and a plurality of flexible sensing legs **46.** Optionally, each of legs **46** has a non-conductive section **48** and a tip **50** in electrical communication with circuit board **44** via a conductive section **52.** In some embodiments of the present disclosure sections **48** and **52** are aligned in a core-shell relationship wherein non-conductive section **48** at least partially surrounds conductive section **52,** but this need not necessarily be the case, since in some embodiments of the present disclosure sections **48** and **52** are aligned in other configurations, such as, but not limited to, a side-by-side configuration.

In some embodiments of the present disclosure one or more of legs **46** does not include separate non-conductive and conductive sections. For example, one or more of legs **46** can have an interlaced structure in a manner that non-conductive regions are interlaced with conductive regions. For example, the leg can be made of a non-conductive material, such as, but not limited to, a non-conductive polymeric material, impregnate with conductive particles, such as, but not limited to, carbon particles or metallic (*e.g.*, nickel) particles.

Preferably, circuit board **44** is detachable from legs **46.** These embodiments are advantageous since they allow making system **46** partially disposable and partially reusable. Specifically, legs **46,** which are in contact with the scalp, can be made disposable, and circuit board **44** can be made reusable. Prior to the mounting of system **10** onto the head of the subject, new disposable legs **46** are attached to circuit board **44** so that it is not necessary to use the same legs for different subjects or different sessions.

Circuit board **44** serves for collecting signals for each individual section **52** and transmitting it to processor **18,** optionally and preferably via a wired bus interface, as further detailed hereinbelow.

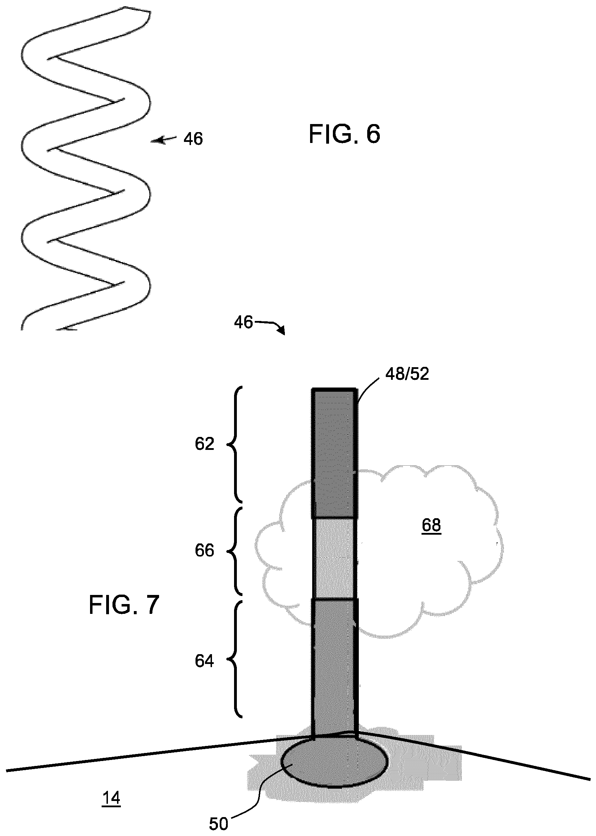

Each leg **46** of system **40** thus enacts one of electrodes **16** of system **10.** Legs **46** can be of any three-dimensional shape that facilitates its bending or collapse towards scalp **14** once wearable body **12** is worn thereon, and once force **42** is applied. FIGs. 3A-5E illustrate a curved shape for legs **46,** wherein the compression force **42** increases the curvature of the shape. Also contemplated are embodiments in which one or more of legs **46,** more preferably each of legs **46,** has a helical shape. A schematic illustration of a leg **46** in these embodiments is illustrated in FIG. 6. In various exemplary embodiments of the invention the shape and orientation of legs **46** is selected that at least one, more preferably each, of leg **46** is oriented along a direction that is non-coincidental with the normal direction to the scalp **14.**

In some embodiments, circuit board **44** is configured for pre-processing the signals sensed by legs **46.** For example, circuit board **44** can include a buffering amplifier and/or be configured for providing active shielding to reduce noise.

It was found by the Inventors that sensing system **40** is capable of penetrating through the hair of subject **22** and also to provide improved contact with scalp **14,** optionally and preferably in dry environment.

Preferably, legs **46** are made, at least in part, of a shape memory material, such as, but not limited to, a shape memory alloy. The shape memory material can be incorporated in the flexible section **48,** in which case the shape memory material is preferably a non-conductive polymer or a conductive material (*e.g.,* a conductive polymer or alloy) that is coated by a non-conductive coating to insulate section **52** from other conductive sections. Alternatively or additionally, the shape memory material can be incorporated in the section **52,** in which case the shape memory material is preferably a conductive polymer or alloy.

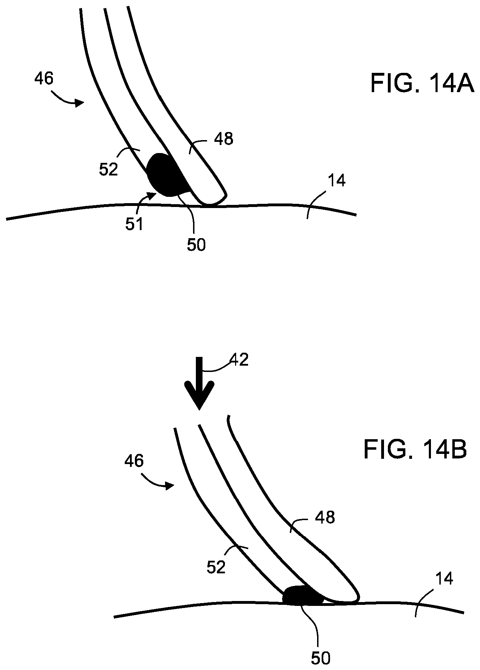

In some embodiments of the present disclosure electrical conductance between scalp **14** and circuit board **44** is established only after force **42** is applied, wherein before the application of force **42** scalp **14** and circuit board **44** are devoid of electrical conductance therebetween. These embodiments are advantageous since they prevent short circuit in case tips of neighboring sensing systems overlap. These embodiments can be realized by ensuring that tip **50** of leg **46** contacts scalp **14** only after force **42** is applied. A preferred configuration for this realization is illustrated in FIGs. 14A and 14B. Shown is a leg **46** in which the conductive **52** and non-conductive **48** sections are aligned in a side-by side configuration. In the absence of force **42** (FIG. 14A), leg **46** is oriented and assumes a shape in which non-conductive **48** section contacts scalp **14** and maintains a gap **51** between tip **50** and scalp **14.** Following the application of force **42** (FIG. 14B), leg **46** experiences a strain and assumes a shape in which the end of conductive section 52 comes in contact with scalp **14.**

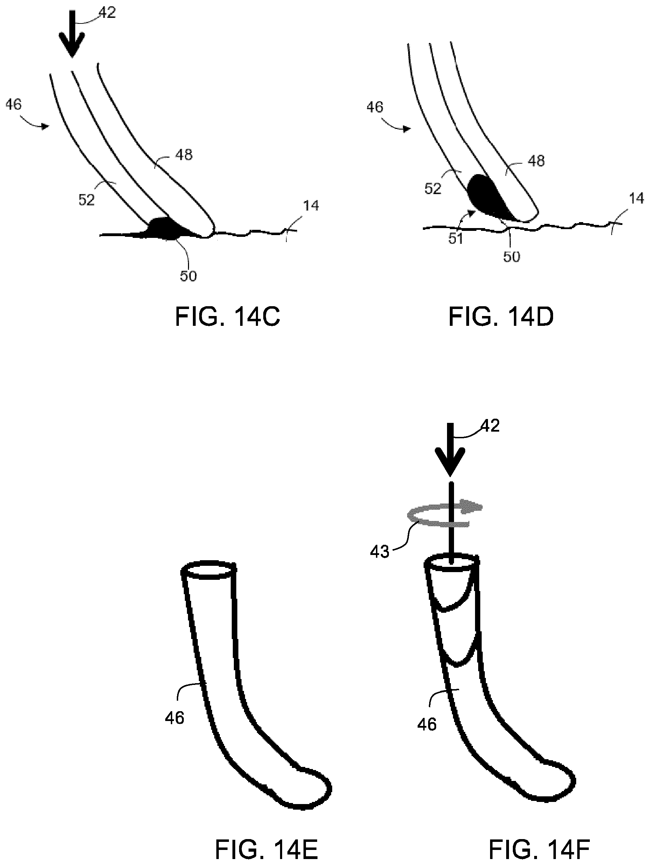

In some embodiments of the present disclosure tip **50** is constructed and designed such that its contact area with scalp **14** is increased after the application of force **42,** as illustrated in FIGs. 14C and 14D. The cross-sectional area of tip **50** in a plane generally parallel to scalp **14** is smaller in the absence of force **42** (FIG. 14D) than in the presence of force **42** (FIG. 14C). The advantage of such a configuration is that it improves the electrode tissue impedance. Preferably, the material of tip **50** is sufficiently soft so that once it is strained by force **42** its surface conforms with the shape of the skin in contact therewith and fills or partially files skin grooves (FIG. 14C).

In some embodiments of the present disclosure legs **46** are arranged such that upon application of compression force **42,** the legs rotate towards the scalp **14.** These embodiments are illustrated in FIGs. 14E and 14F, showing leg **46** before (FIG. 14E) and after (FIG. 14F) the application of force **42,** wherein leg **46** is arranged to rotate **43** in response to force **42.** The rotation can be effected by means of the arrangement of legs **46** over a base of a body **54** of system **40** (not shown in FIGs. 14A-D, see FIGs. 3A-5F). For example, the legs can be arranged in a spiral manner so that once force **42** is applied, the spiral arrangement ensures transforming the inwardly applied force into a turning force. Alternatively or more preferably additionally, force **42** is applied while establishing rotary motion to legs **46.** The conductive **52** and non-conductive **48** sections of leg **46** are not shown in FIGs. 14E and 14F, but the ordinarily skilled person, provided with the information described herein, would know how to construct leg **46** to include the conductive **52** and non-conductive **48** sections.

Combination of the embodiments illustrated in FIGs. 14A-B and/or FIGs. 14C-D with the embodiments illustrated in FIGs. 14E-F are also contemplated. In this combination of embodiments, force **42** both causes leg **46** to rotate and applies a strain on leg **46** so that tip **50** comes in contact with scalp **14.**

Also contemplated are embodiments in which a separate force is applied for rotating the leg.

Preferably, the tip **50** is made of soft material with low shore (e.g., Shore OO Hardness of from about 10 to about 100), that experiences a strain once force **42** is applied, as illustrated in FIGs. 14B and 14C. These embodiments are useful from the standpoint of user's comfort and are also advantageous since the strain increases the footprint on scalp **14.**

Shore OO Hardness describes a material's resistance to permanent indentation, defined by type OO durometer scale. Shore A hardness is typically determined according to ASTM D2240-00.

A representative example of a material from which tip **50** can be made include, without limitation, silicon coated with AgCl, or Polyurethane composites with metal, or Carbon Black, but other conductive materials are also contemplated.

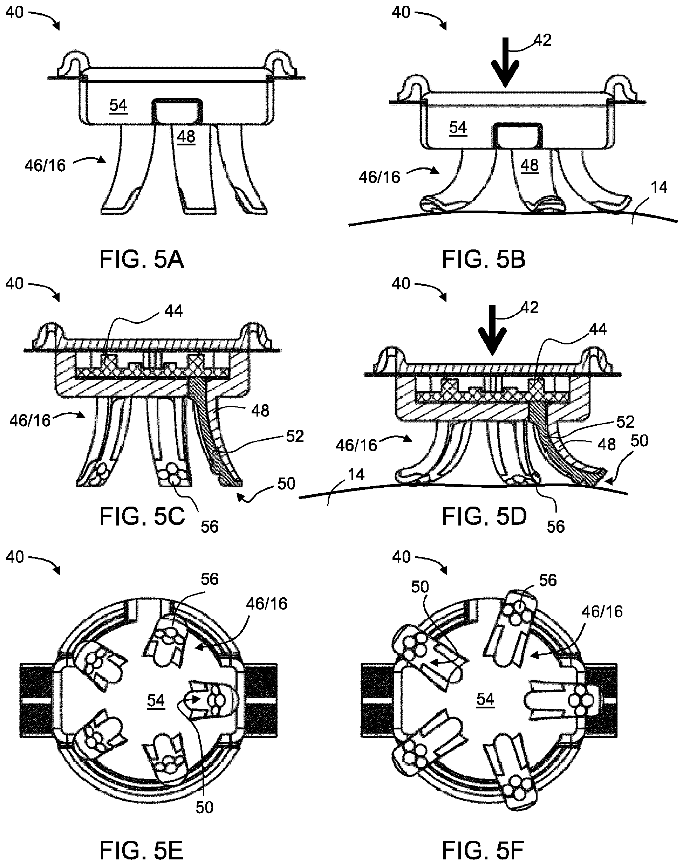

In some embodiments of the present disclosure tip **50** is constructed and designed such that a friction force between tip **50** and scalp **14** is sufficient to prevent movement of tip **50** from its location after the application of force **42.** Typically, but not necessarily, tip **50** is coated with a coating having a plurality of micrometric or nanometric structures **56** (FIGs. 5C-F), so as to increase the friction force and also increase the surface area of tip **50** hence to improve the electrical conductivity of tip **50.**

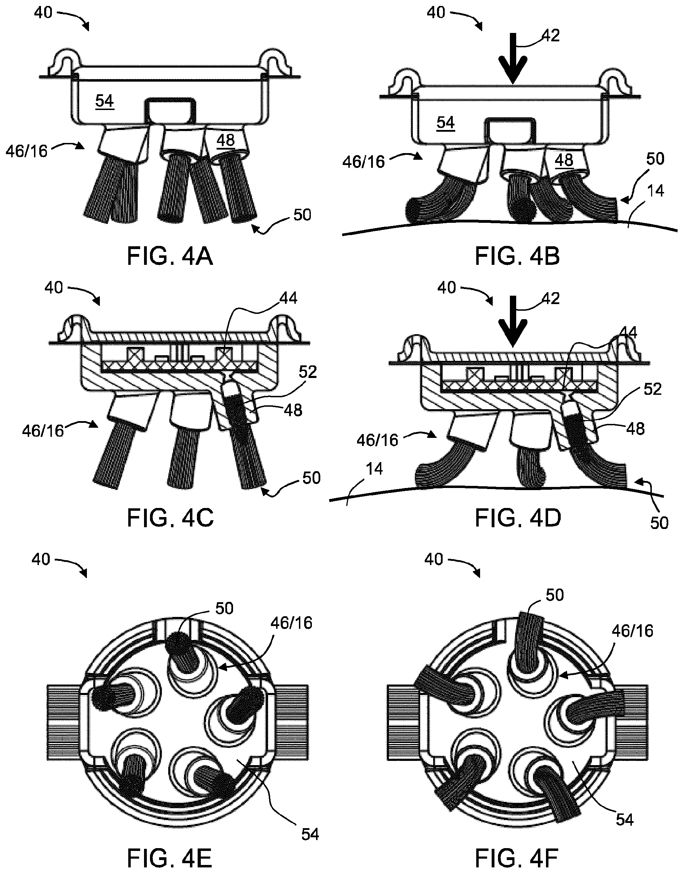

The present embodiments contemplate several types of conductive sections for legs 46. In some embodiments of the present disclosure the conductive section **52** of one or more of legs **46** is a metallic wire. These embodiments are illustrated in FIGs. 3A-F. In some embodiments of the present disclosure the conductive section **52** of one or more of legs **46** comprises a bundle of conductive bristles. These embodiments are illustrated in FIGs. 4A-F. In some embodiments of the present disclosure the conductive section **52** of one or more of legs **46** is made of a conductive polymer. These embodiments are illustrated in FIGs. 5A-F. Combination of these embodiments, *e.g.,* a sensing system with at least two legs **46** with different types of sections, are also contemplated.

Referring in particular to FIGs. 4A-F, each bristle of section **52** optionally and preferably have a thickness of from about 0.05 mm to about 0.4 mm, *e.g.,* 0.15 mm. The advantage of having bundle of conductive bristles for section **52** is that it facilitates penetration into the hair of the subject. Another advantage is that the tip **50** of the bundles conforms with the shape of scalp **14,** whereby different bristles of the bundle may engage points scalp **14** that have different heights resulting in enlarged footprint of system **40** on the scalp. In some embodiments of the present disclosure bundles are arranged in a spiral manner (FIGs. 4E and 4F) over a base of a body **54** of system **40,** so as to prevent bristles of one bundle to contact with bristles of another bundle. Once force **42** is applied, the spiral arrangement ensures transforming the inwardly applied force into a turning force that acts as a screw on the tip of the bristles. The advantage of using the bristles is that it reduces the likelihood that the electrodes will be entangled with the subject's hair.

Reference is now made to FIG. 7 which is a schematic illustration of a leg **46** of system **40,** in embodiments in which the non-conductive section **48** and/or the conductive section **52** of leg **46** comprises a hydrophobic zone **62** and a hydrophilic zone **64.**

Preferably, hydrophobic zone **62** is at the upper part of leg **46** (not in contact with the scalp) and hydrophilic zone **64** is at the lower part of leg **46** (adjacent to tip **50**). The advantage of this embodiment is that humidity, resulting, *e.g.*, from sweat at the surface of scalp **14,** is concentrated at the vicinity of the lower part of leg **46** increasing the conductivity with the human tissue, and prevents it from arriving at the circuit board **44** (not shown). This embodiment is also advantageous since it prevents electricity shorts between adjacent sensor systems.

In some embodiments of the present disclosure leg **46** also comprises an intermediate zone **66** between hydrophobic zone **62** and hydrophilic zone **64.** Intermediate zone **66** is less hydrophobic than hydrophobic zone **62,** and less hydrophilic than hydrophilic zone **64.** For example, intermediate zone **66** can be neither hydrophilic nor hydrophobic. In some embodiments the hydrophobicity of intermediate zone **66** is gradually increased in the upward direction. The advantage of having intermediate zone **66** is that the temperature differences between scalp **14** and wearable body **12** create a sink at the vicinity of zone **64.** Since the temperature is typically higher at scalp **14** than at wearable body **12,** air vapors **68** tends to condense at the higher parts of leg **46.** Since the upper part is hydrophobic, the vapor **68** condenses at a region on the intermediate zone **66** and be drawn downwards by the hydrophilic zone **64** acting as a vapor sink. This prevents electrical shorting between adjacent legs of the same sensing system and/or between adjacent legs of adjacent sensing systems.

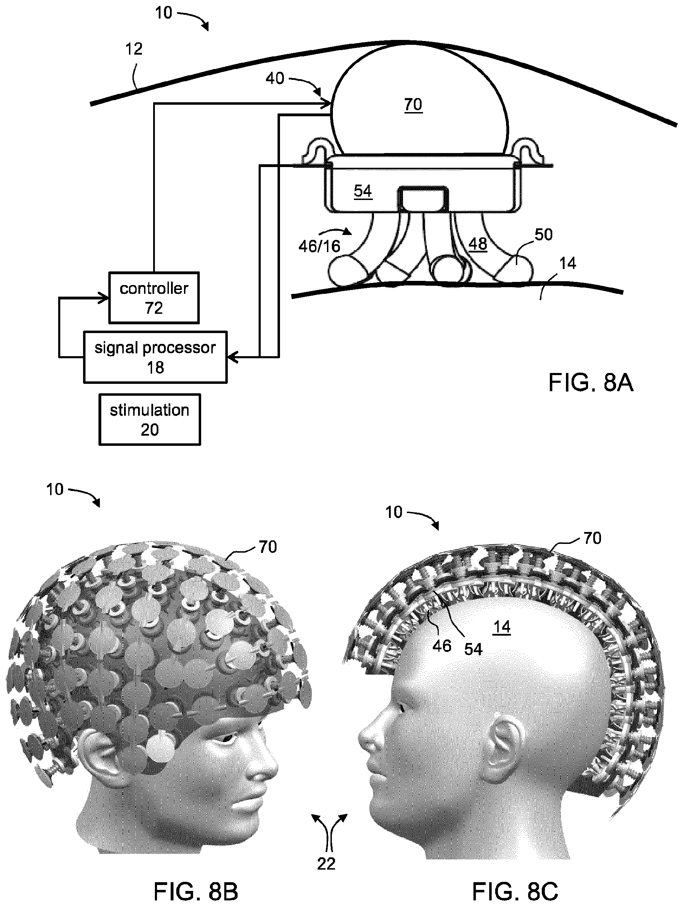

FIGs. 8A-H are schematic illustration of system **10** in embodiments of the disclosure in which system **10** comprises controllable actuators for applying force to the electrodes. In the illustrations shown in FIGs. 8A-H, which is not to be considered as limiting, the electrodes of system **10** are grouped into sensing systems **40,** but this need not necessarily be the case, since configurations in which the electrodes are not grouped are also contemplated, according to some embodiments of the present invention. For clarity of presentation, only one sensing system **40** is shown in FIG. 8A, but it is to be understood that many sensing systems like system **40** can be incorporated in EEG system **10.**

In the illustrated embodiment, system **10** comprises a plurality of controllable actuators **70,** for applying force to electrodes **16** or legs **46.** For clarity of presentation, one actuator is magnified in FIG. 8A. Actuators **70** are designed and configured to apply to electrodes **16** or legs **46** a compression force (*e.g.,* force **42**), optionally and preferably a non-periodic compression force. Actuators **70** can in some embodiments of the present disclosure be designed and configured to apply to electrodes **16** or legs **46** a periodic (*e.g*., sinusoidal) force so as to vibrate the legs or electrodes or to generate a hammering effect, facilitating better penetration of the electrodes or legs through the and better spread of the contact area between the tips and the scalp. A hammering effect can be generated by controlling actuator **70** to periodically apply and terminate the application of a compression force to the electrodes. Actuators **70** can in some embodiments of the present invention be designed and configured to apply to electrodes **16** or legs **46** a rotating force so as to rotate the legs, for example, about a longitudinal axis of the legs, as illustrated in FIG. 14F.

Preferably, but not necessarily, each actuator **70** is mounted on top of the body **54** of sensing system **40.**

Further contemplated, are combinations of the above embodiments. For example, one or more actuators can apply a compression force (*e.g.,* force **42**), and one or more actuators can apply a periodic force to vibrate the legs or to generate a hammering effect.

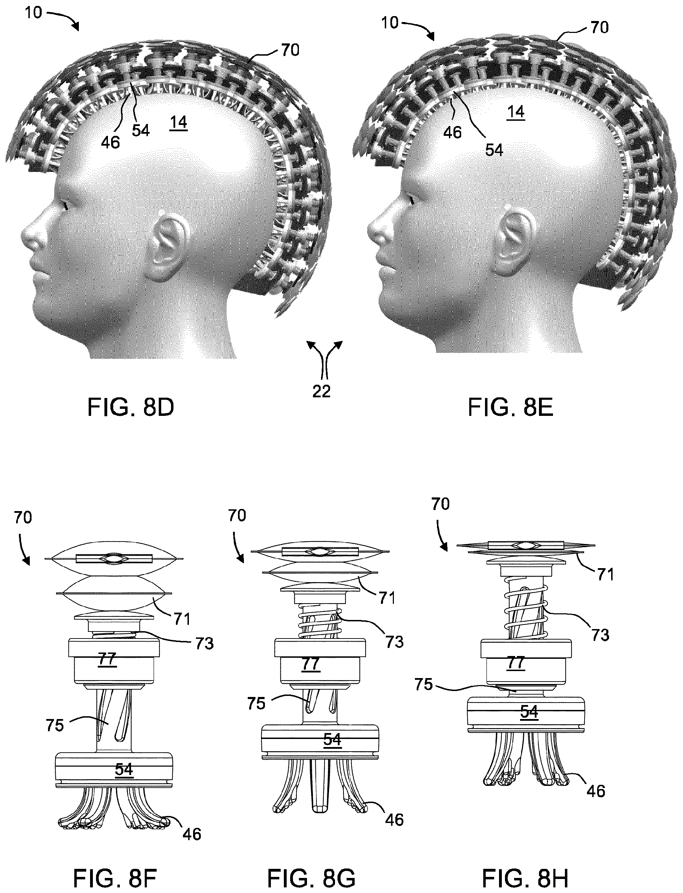

FIGs. 8B-E schematically illustrate system **10** once mounted on scalp **14** of a subject **22,** FIG. 8B is a perspectives view, and FIGs. 8C, 8D and 8E are cross-sectional views, at three different states of actuators **70.** Specifically, in FIG. 8C actuators **70** are in a relaxed state in which no force is applied to legs **46,** in FIGs. 8D and 8E actuators **70** are apply a force to legs **46,** wherein the state shown in FIG. 8E correspond to a stronger force compared to the state shown in FIG. 8D, thereby bringing body **54** closer to scalp **14** and applying more strain to legs **46** creating higher footprint areas between the tips and the scalp.

Each of actuators **70** can be embodied, for example, as inflatable balloon, applying force upon inflation thereof, a piezoelectric actuator or an electric actuator (*e.g.,* a solenoid) or motor applying force upon application of voltage thereto, a pneumatic actuator applying force by means of a motion of a piston, *etc.*

FIGs. 8F-H schematically illustrate actuator **70** in embodiments in which actuator **70** comprises an inflatable balloon **71.** In these embodiments, actuator **70** can also comprises a retraction mechanism **73,** which can be embodied as an elastic member, *e.g.,* a spring, a rotating shaft **75** and an annular stator **77.** Shaft **75** is positioned within stator **77.** The proximal end of shaft **75** engages balloon **71** and the distal end of shaft **75** is connected to body **54.** Preferably, shaft **75** is provided with one or more helical grooves and the inner surface of stator **77** is provided with one or more protrusions fitting into the grooves of stator **77,** so that stator **77** and shaft **75** serve as a push-to-rotate mechanism. Upon inflation of balloon **71,** shaft **75** is pushed through stator **77** in the direction of the scalp (not shown in FIGs. 8F-H) and is being rotated by stator **77.** Body **54,** which is connected to the distal end of shaft **75,** rotates while applying the force on legs **46.** Once balloon **71** is deflated, retraction mechanism **73** retracts shaft **75** through stator **77.**

FIGs. 8F-H illustrate three states of actuator **70.** FIG. 8F illustrates a state at which the force applied to legs **46** is sufficiently high to strain the legs, FIG. 8G illustrates a state at which the force applied to shaft **75** is sufficient to establish contact between legs **46** and the scalp, and FIG. 8H illustrates a state at which shaft **75** is fully retracted by mechanism **73,** until legs **46** do not contact the scalp. A typical inflation pressure *P*_{c} to establish contact between legs **46** and the scalp (FIG. 8G) is a differential pressure (relative to the ambient pressure) of from about 2 to about 12, *e.g.,* about 4 PSI. When the differential pressure within balloon **71** is above P_{c} legs **46** are pressed against the scalp and experience strain (FIG. 8F), and when the differential pressure within balloon **71** is less than P_{c} shaft **75** is retracted by mechanism **73** (FIG. 8H). Other pressure levels are also contemplated. In various exemplary embodiments of the invention the pressure within balloon **71** at the state at which legs **46** are pressed against the scalp is selected such that the load of legs **46** on the scalp is not more than an empirical parameter (e.g. 3 N/cm²).

When actuator **70** of system **40** comprises balloon **71,** shaft **75** and stator **77,** wearable body **12** optionally and preferably comprises an outer shell **12a** and an inner shell **12b.** FIGs. 9A and 9B showing an exploded view (FIG. 9A), an assembled view (FIG. 9B) of system **10.** In the illustrated embodiments, outer shell **12a** serves for supporting balloon **71** and pneumatic tubes **162** for inflating and deflating balloon **71,** and inner shell **12b** serves for supporting other components of sensing system **40,** including at least stator **77,** circuit board **44,** and body **54** with legs **46.** Optionally and preferably, at least one of shells **12a** and **12b,** more preferably both shells **12a** and **12b,** is rigid. Preferably, the size of inner shell **12b** is adjustable to fit onto scalp **14.**

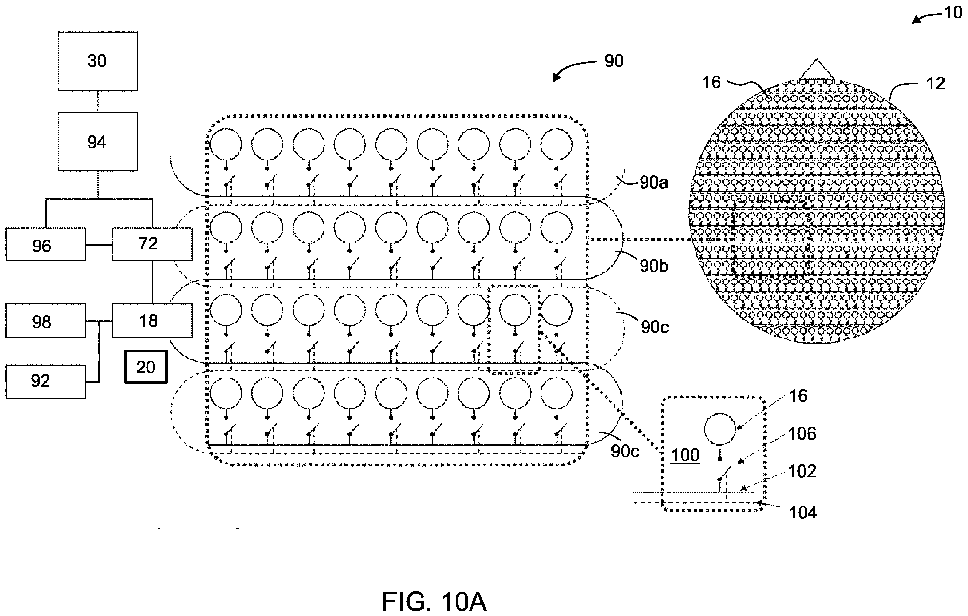

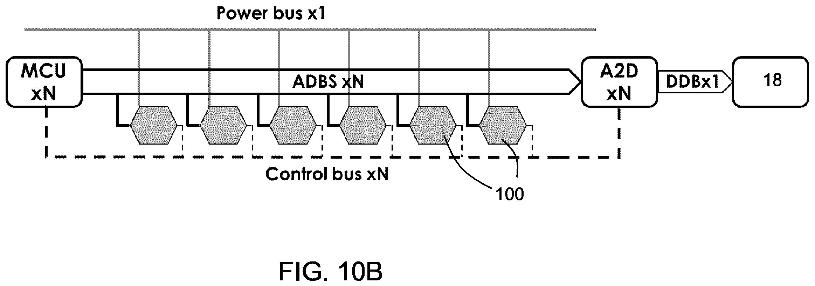

Typically, system **10** also comprises a wired bus interface **90** for establishing electrical contacts among various components of system **10.** Interface **90** can be mounted on inner shell **12b.** A more detailed description of a bus interface suitable for the present embodiments is provided hereinunder with reference to FIGs. 10A and 10B.

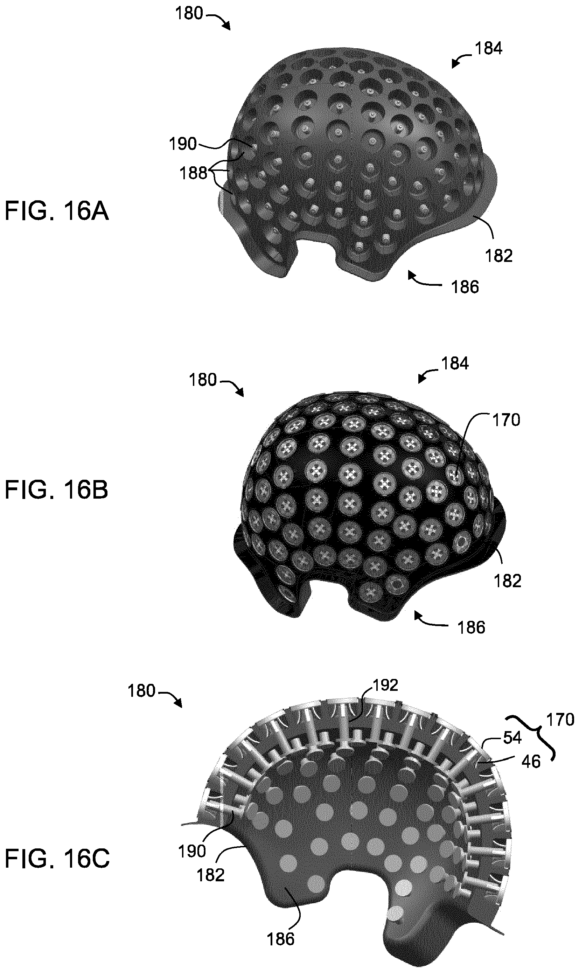

Circuit board **44** is preferably detachable from body **54,** and can be enclosed in a housing **164,** designed for receiving body **54.** In some embodiments of the present invention housing **164** comprises a rigid wall **165** and a flexible membrane **167** connecting wall **165** to shaft **75.** The advantage of these embodiments is that they provide flexibility in the orientation of housing **164** with respect to shaft **75,** hence to allow the sensing systems **40** to conform with the shape of the scalp. Male and female mating snap members **166** are optionally and preferably employed for facilitating fast connection and detachment between circuit board **44** and body **54.** A preferred technique for connecting body **54** to housing **164** is by means of a jig system, as described hereinunder with reference to FIGs. 16A and 16B.

In some embodiments of the present disclosure bodies **54** of two or more of, optionally and preferably all of, sensing systems **40** are interconnected by means of a sheet member **168,** which is optionally and preferably shaped as a cap. Typically, bodies **54** are embedded within sheet member **168.** A perspective view of sheet member **168** (shaped as a cap), with interconnecting sensing systems **40** embedded therein is illustrated in FIG. 9C. Sheet member **168** is preferably made flexible to provide each sensing system **40** with independent rotational and translational motion. In some embodiments of the present disclosure bodies **54** are securely connected to member **168** to form a unitary structure that cannot be disassembled by the end user without causing a physical damage (*e.g*., rupture, breakage). Optionally and preferably, bodies **54** and member **168** form a disposable and non-disassemblable structure.

FIG. 9B illustrates an exemplified situation in which the balloon **71** of one sensing system is inflated to ensure contact between the legs **46** and the scalp **14,** and the balloon **71** of an adjacent sensing system is deflated so that there is no contact between the legs **46** and the scalp **14.** Body **54** with legs **46** and optionally also flexible member **168** are collectively referred to herein as electrode assembly **170.**

Referring again to FIG. 8A, system **10** optionally and preferably comprises a controller **72** configured for individually controlling each actuator **70** to apply the force. Controller **72** receives control signals from signal processor **18.** Signal processor **18** can signal controller **72** to operate actuator **70** following a user input, for example, when the subject **22** feels discomfort, the subject **22** can enter a command to processor **18** to release the pressure on the electrodes or to initiate a vibration protocol. Alternatively or additionally, signal processor **18** and controller **72** operate in a closed loop control, wherein signal processor **18** receives and processes EEG signals from the electrodes (*e.g.,* legs **46** or electrodes **16** of the sensing systems **40**) and transmits the control signals to controller **72** based on the processing.

Typically, signal processor **18** determines electrode-tissue impedance or one or more signal quality measures and transmits the control signals to controller **72** based on the determined impedance.

As used herein "electrode-tissue impedance" (ETI) is a ratio of the voltage drop between two given electrodes to the current flowing between those two electrodes.

For example, signal processor **18** can signal controller **72** to increase a compressive force applied by actuator **70** when the determined impedance is above a predetermined threshold, and release it otherwise. This allows system **10** to operate at low impedance values at all times. Controller **72** can receive control signals for controlling actuator **70** also when actuator **70** vibrates the electrodes or generates a hammering effect. Typically, signal processor **18** signals controller **72** to initiate a vibration or hammering protocol by actuator **70** when the determined impedance is above a predetermined threshold.

The advantage of executing vibration and/or hammering protocol is that it allows to relocate the sensing system and to improve comfort to the user.

Signal processor **18** can also receive from actuator **70** signals indicative the force or pressure it applies to the electrodes or legs. For example, when actuator **70** comprises an inflatable balloon, signal processor **18** can receive from actuator **70** signals describing the pressure within the balloon. The signals that are indicative of the force or pressure can be used by signal processor **18** in selecting the control signals it transmits to controller **72.** In an embodiment of the disclosure, signal processor **18** signals controller **72** to increase a compressive force applied by actuator **70,** in response to a determination that (i) the determined impedance is above the predetermined, and (ii) that the applied force is below a predetermined threshold; and to initiate a vibration or hammering protocol by activation actuator **70** to apply a periodic force, in response to a determination that (i) the determined impedance is above the predetermined, and (ii) that the applied force is equal to or above the predetermined threshold. A detailed procedure suitable for implementing a closed loop control is provided below.

Reference is now made to FIG. 10A which is a schematic illustration of system **10** in embodiments of the disclosure in which signal transmission among various components of system **10** is via bus architecture. Sown in FIG. 10A are wearable body **12,** electrodes **16,** processor **18,** stimulation system **20,** and controller **72** as further detailed hereinabove. Further illustrated is a wired bus interface **90.** Preferably, bus interface **90** comprises a plurality of bus rows **90a, 90b,** *etc.,* each distributing signals from and to several electrodes on wearable body **12** (*e.g.,* a row of electrodes). Also illustrated is power source **30** and a power block **94** that distributes power to the other components of system **10,** a human machine interface **96** for allowing the operator to enter commands, select parameters, or otherwise reconfigure controller **72** and/or processor **18,** a wire data interface **98** via which communication is established between processor **18** and bus interface **90.** A communication system **92** employing a communication technique, such as, but not limited to, Wi-Fi, Bluetooth^{®}, wireless telephony or the like may also be included in system **10** for allowing system **10** to communicate, via wire data interface **98,** with an external system, such as a computer, a cloud computing facility, a cloud storage facility or the like.

For each of the rows of bus interface **90,** all the corresponding electrodes share same galvanic bus wire, and the EEG data is gated by controller **72.** With specific reference to FIG. 10A, each of the rows of bus interface **90** comprises a data bus **102,** a control bus **104,** and a plurality of bus cells **100.** The data bus **102** and control bus **104** are shared by all the electrodes corresponding to the respective bus row. The data bus **102** can be an analog or digital data bus, depending whether digitization is executed before or after transmission of the EEG signals to processor **18.** Each cell **100** is addressable by controller **72** and comprises a control gate **106** that individually controls the connection state of a specific electrode **16.** Optionally and preferably each electrode publishes over the bus row a digital identification for allowing controller **72** to identify the bus cell that controls the respective electrode. Controller **72** specifically addresses the respective bus cell **100** and transmits gating signals to switch the respective electrode between a connected and unconnected state.

When it is desired to collect EEG signals globally from a region-of-interest over the scalp, controller **72** electrically groups the electrodes that morphologically cover or able to sense signals from the region-of-interest, by transmitting gating signals to the respective cells, wherein the desired electrodes are switched, in a parallel manner, to a connected state. The signals from the electrodes that morphologically cover or able to sense signals from the region-of-interest are combined, optionally and preferably coherently, thereby allowing magnification of the signal collected from the region-of-interest. Controller **72** can electrically group electrodes also when processor **18** determines, based on signal processing analysis, that the quality of the signal is reduced, for example, based on the electrode-tissue impedance or signal-to-noise ratio. For example, when processor **18** determines that the impedance at electrodes that are within a specific area over the scalp is above a predetermined threshold, processor **18** can signal controller **72** to electrically group those electrodes. When processor **18** determines that the impedances at electrodes that cover several distinct areas over the scalp are above a predetermined threshold, processor **18** can signal controller **72** to electrically group each of those electrodes to one or more neighboring electrodes.

FIG. 10B is a schematic illustration of the architecture of a single (out of N) row bus, according to some embodiments of the present disclosure. A power bus provides power to all the bus cells **100.** The power bus can be shared by all the row buses. A main control unit MCU, which is a part of controller **72** (FIG. 10A) that is allocated to the specific row bus, optionally issues via the control bus commands for scanning, grouping, sampling and data retrieval based on the electrode's digital identification. The data bus optionally and preferably comprises an analog data bus (ADBS), which can in some embodiments of the present disclosure gather the raw analog EEG signals to an analog to digital (A2D) converter. The raw sampled signal may be of single electrodes or a group of electrodes, if electrode grouping is employed. The main control unit can use the control bus to synchronize the sampling and direct the sampled signals from the electrodes to the A2D converter. From the A2D converters a digital data bus (DDB) optionally and preferably gathers all EEG data to processor **18.**

One advantage of bus interface **90** (FIG. 10A) is that it reduces the need for individual cables that would otherwise be required for each electrode, thereby reducing the weight and complexity of the system. Another advantage of bus interface **90** is that the mapped location of the electrodes allows simple routing of orders and data retrieval to and from electrodes according to specific neurological event in the brain. In various exemplary embodiments of the invention system **10** employs high rate electronics (e.g., sampling rate of at least 200Hz or at least 300Hz or at least 400Hz or at least 500Hz, with word length of at least 32-bit or at least 64-bit). This allows system **10** to switch and sample each electrode at the required EEG frequency band, while digitally sampling EEG signals by grouping electrodes per dedicated assignments. Another advantage of bus interface **90** is that it allows multi drop reading of the electrode, wherein readouts can be collected serially using the same bus channel. Another advantage of bus interface **90** is that the power management is improved, since it is not necessary to activate all electrodes at the same time.

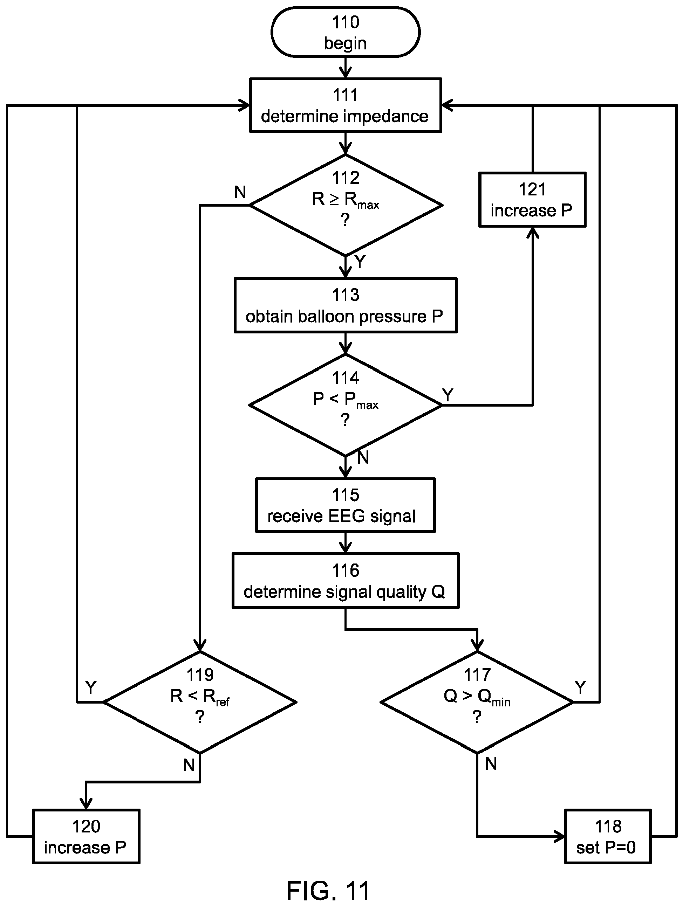

FIG. 11 is a flowchart diagram describing a close loop procedure for operating an actuator to apply a compression force on an electrode, according to some embodiments of the present disclosure. The procedure can be executed, for example, by system **10,** particularly by actuator **70,** electrodes **16,** processor **18** and controller **72.** The procedure begins at **110** and continues to **111** at which the electrode-tissue impedance is determined, for example, by signal processor **18.** The procedure continues to decision **112** at which the impedance is compared to a first predetermined threshold Rₘₐₓ.

A representative example of a value for the first predetermined threshold Rₘₐₓ is from about 50 KΩ to about 250 KΩ.

If the impedance is less than Rₘₐₓ, the procedure continues to decision **119** at which the impedance is compared to a second predetermined threshold R_{ref}.

A representative example of a value for the second predetermined threshold R_{ref} is from about 60 KΩ to about 180 KΩ.

If the impedance is above or equal to R_{ref} the procedure proceeds to **120** at which a compression force applied to the electrode is increased. From **120** the procedure loops back to **111.**

If, at decision **112,** the impedance is above or equal to Rₘₐₓ, the procedure continues to **113** at which a value of a parameter indicative of the force applied by the actuator is obtained. For example, when the actuator comprises a balloon, the procedure can obtain the pressure within the balloon. The procedure continues to decision **114** at which the parameter (pressure, in the present example, preset as comfortability of user threshold) is compared to a predetermined threshold Pₘₐₓ indicative of an upper limit of the force that is to be applied (upper limit of a pressure, in the present example).

A representative example of a value for the predetermined parameter threshold Pₘₐₓ, is from about 25 cmH₂O to about 500 cmH₂O, e.g., about 250 cmH₂O.

If the parameter is below the Pₘₐₓ, the procedure continues to **115** at which an EEG signal is received from the electrodes and to **116** at which the quality of the signal is determined. This is optionally and preferably done by processor **18,** and may include calculating at least one of the impedance, the power and the frequencies in the signal.

The procedure continues to decision **117** at which the quality of the signal is compared to a quality threshold or a set of quality thresholds. For example, data processor **18** can calculate a score Q using the calculated quantities and compares the score to a predetermined score threshold Qₘᵢₙ.

For example, when the score Q is defined over a scale between 0 and 1, a representative example of a value for a predetermined score threshold Qₘᵢₙ, is from about 0.5 to about 0.9, e.g., about 0.7.

Representative examples for signal quality parameters that can be used for calculating the score Q include, without limitation, electrode-tissue impedance, proportion of artifacts in the time domain, signal-to-noise ratio in the time domain, parietal alpha-wave power in the frequency domain, frontal theta-wave power effect in the frequency domain, and parietal alpha-wave demanding cognitive tasks power in the frequency domain. In some embodiments of the present invention one or more, optionally and preferably each, of these parameters is subjected to thresholding and the results of the thresholding can be used for calculating the score Q.

A typical threshold for the artifacts in the time domain parameter, when calculated for the overall record duration, is from about 18% to about 22%, *e.g.,* about 20%, wherein a signal for which the value of this parameter is below or equals the threshold is assigned with a score that is higher than a signal for which the value of this parameter is above the threshold.

The signal-to-noise ratio in the time domain parameter can be calculated in units of dB according to the formula SNR=IOlog₁₀(σₓ²/σₑ²), where σₓ² and σₑ² are, respectively, the variances of the signal and the noise. A typical threshold for this parameter is from about -2dB to about 0 dB, *e.g.,* about -1dB, wherein a signal for which the value of this parameter is above or equals the threshold is assigned with a score that is higher than a signal for which the value of this parameter is below the threshold.

A typical threshold for the parietal alpha-wave power in the frequency domain parameter is from about 3% to about 9%, *e.g.,* about 6%, wherein a signal for which the value of this parameter is above or equals the threshold is assigned with a score that is higher than a signal for which the value of this parameter is below the threshold. A typical threshold for the frontal theta-wave power in the frequency domain parameter is from about 1% to about 5%, *e.g.,* about 3%, wherein a signal for which the value of this parameter is above or equals the threshold is assigned with a score that is higher than a signal for which the value of this parameter is below the threshold. A typical threshold for the parietal alpha-wave demanding cognitive tasks power in the frequency domain parameter is from about 1% to about 5%, *e.g.,* about 3%, wherein a signal for which the value of this parameter is above or equals the threshold is assigned with a score that is higher than a signal for which the value of this parameter is below the threshold.

If the quality is above the quality threshold, the procedure loops back to **111.** If, at decision **117,** the quality is below the quality threshold, the procedure continues to **118** at which the force is set to zero, for example, by terminating a previously applied force. From **117** the procedure loops back to **111.**

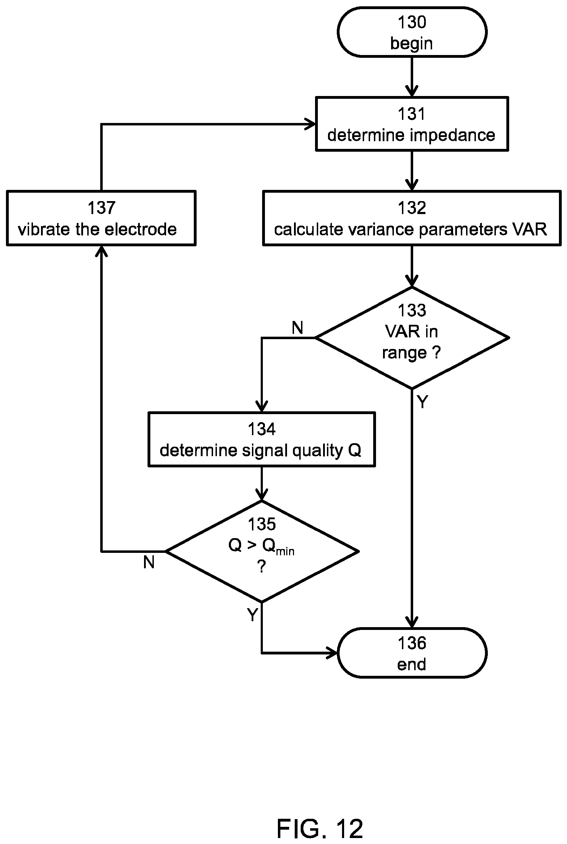

FIG. 12 is a flowchart diagram describing a close loop procedure for operating an actuator to vibrate the electrodes, according to some embodiments of the present disclosure. The procedure can be executed, for example, by system **10,** particularly by actuator **70,** electrodes **16,** processor **18** and controller **72.** The procedure begins at **130** and continues to **131** at which the electrode-tissue impedance is determined, for example, by signal processor **18.** The procedure continues to **132** at which variance parameters are calculated, *e.g.,* by signal processor **18,** among signals received from a plurality of the electrodes. The procedure continues to decision **133** at which the calculated VAR is compared to a predetermined variance range. Typically the predetermined variance range is chartered by a lower variance limit V_{L} and an upper variance limit Vu.

A representative example of impedance value for the lower variance limit V_{L} is from about 5KΩ to about 100KΩ, e.g., about 75KΩ. A representative example of impedance value for the upper variance limit Vu is from about 250KΩ to about 500KΩ, e.g., about 350KΩ.

If the calculated VAR is within the range (*e.g.*, V_{U}>VAR>V_{L}), the procedure continues to **136** at which it ends. If the calculated VAR is outside the range (*e.g.*, VAR>V_{U} or VAR<V_{L}), the procedure continues to **134** at which the quality of the signal is determined. This is optionally and preferably done by processor **18** and may include calculating at least one of the power and the frequencies in the signal, signal to noise ratio and artifacts percentage. The procedure continues to decision **135** at which the quality of the signal is compared to a quality threshold or a set of quality thresholds. For example, data processor **18** can calculate a score Q using the calculated quantities and compares the score to a predetermined score threshold Qₘᵢₙ, as further detailed hereinabove. If the quality is above the quality threshold, the procedure continues to **136** at which the procedure ends. If, at decision **135,** the quality is below the quality threshold, the procedure continues to **137** at which the actuator is operated to initiate vibration. From **137** the procedure loops back to **131.**

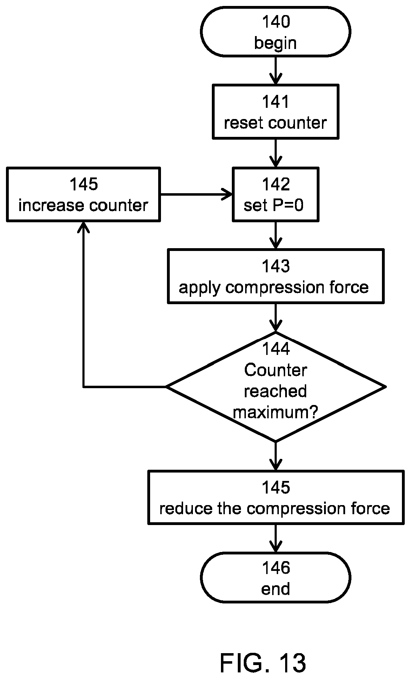

FIG. 13 is a flowchart diagram describing a close loop procedure for operating an actuator to provide a hammering effect, according to some embodiments of the present disclosure. The procedure can be executed, for example, by system **10,** particularly by actuator **70,** electrodes **16,** processor **18** and controller **72.** The procedure begins at **140** and continues to **141** at which a counter is reset. The procedure continues to **142** at which the force applied by the actuator is terminated. If there is no force that is applied at the beginning **140** of the procedure, operation **141** can be skipped. When the actuator comprises a balloon, operation **141** include reducing the pressure in the balloon to the ambient pressure. The procedure continues to **143** at which the actuator is operated to apply compression force at a predetermined magnitude and predetermined profile in the time domain (*e.g.* ramp, Sine, step, *etc*.). For example, when the actuator comprises an inflatable balloon, the balloon is inflated to a predetermined pressure. In some embodiments of the present invention the force applied at **143** is the maximal allowed force. At decision **144** the value of the counter is compared to a predetermined maximum count parameter. If the counter does not reach the maximum, the counter is increased **145** by 1, and the procedure loops back to **142.** If the counter reaches the maximum, the procedure continues to **146,** at which the magnitude of the force is reduced to a predetermined reference level, and to **146** at which the procedure ends.

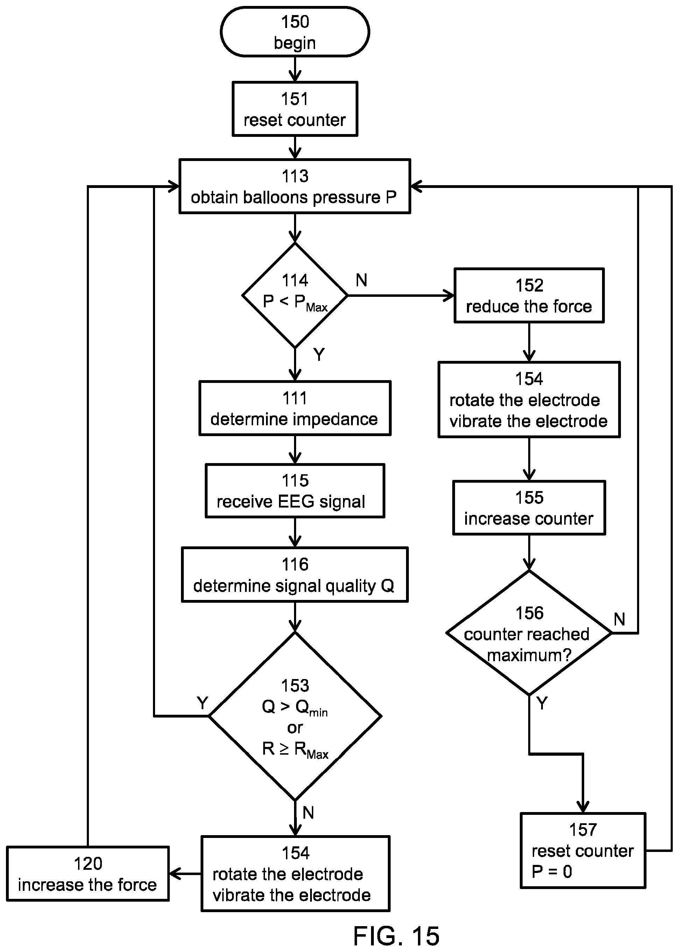

FIG. 15 is a flowchart diagram describing a combined procedure for operating an actuator, according to some embodiments of the present disclosure. The procedure can be executed, for example, by system **10,** particularly by actuator **70,** electrodes **16,** processor **18** and controller **72.** The procedure begins at **150** and continues to **151** at which a counter is reset. The procedure continues to **113** at which a value of a parameter indicative of the force applied by the actuator is obtained, as further detailed hereinabove. The procedure continues to decision **114** at which the parameter is compared to a predetermined threshold Pₘₐₓ as further detailed hereinabove. If the parameter is below Pₘₐₓ, the procedure continues to **111** at which the electrode-tissue impedance is determined, as further detailed hereinabove. If the parameter is above or equal to Pₘₐₓ, the procedure continues to **152** at which the applied force is reduced.

From **111** the procedure optionally and preferably continues to **115** at which an EEG signal is received from the electrodes and to **116** at which the quality of the signal is determined, as further detailed hereinabove. At decision **153** the quality of the signal is compared to a quality threshold or a set of quality thresholds, and the impedance is compared to a first predetermined threshold Rₘₐₓ, as further detailed hereinabove. If at least one of the quality of the signal (according to one or more of the measures described herein) and the impedance satisfy the respective criterion at **153** the procedure loops back to **113.** Otherwise, the procedure continues to **154** at which the electrode is rotated and vibrated, and to **120** at which the force is increased as further detailed hereinabove.

From **154** the procedure loops back to **113.**

From **152** the procedure continues to **154** at which the electrode is rotated and vibrated. In some embodiments of the present invention **154** is executed by means of a reciprocal activation of the aforementioned push-to-rotate mechanism embodied as stator **77** and shaft **75.** Specifically, force **42** is applied and released repeatedly so that the electrode is rotated in one direction when the force is applied and in the opposite direction when the force is released.

At **155** the counter is increased by 1, and at decision **156** the value of the counter is compared to a predetermined maximum count parameter. If the counter does not reach the maximum, the procedure loops back to **113.** If the counter reaches the maximum, the procedure continues to **157,** at which the counter is reset and the force is set to zero, as further detailed hereinabove. From **157** the procedure loops back to **113.**

Reference is now made to FIGs. 16A-D which are schematic illustrations of a jig system **180** according to some embodiments of the present invention. Jig system **180** is particularly useful for connecting electrode assembly **170** with housing **164** of sensing system **40** to establish electrical communication between legs **46** and circuit board **44.** EEG system **10** and jig system **180** can be distributed to users as a kit. The kit can also include one or more sets of disposable electrode assemblies **170** of sensing system **40** each electrode assembly **170** may include body **54** carrying legs **46** as further detailed hereinabove.

Jig system **180** can comprise a scaffold **182** having an outer surface **184** and an inner surface **186.** Outer surface **184** is designed and constructed to fittedly receive inner shell **12b** of system **10** (not shown in FIGs. 16A-C, see FIGs. 9A-C), and is formed with a plurality of recesses **188** each being size-wise and shape-wise compatible with disposable electrode assembly **170** of sensing system **40.**

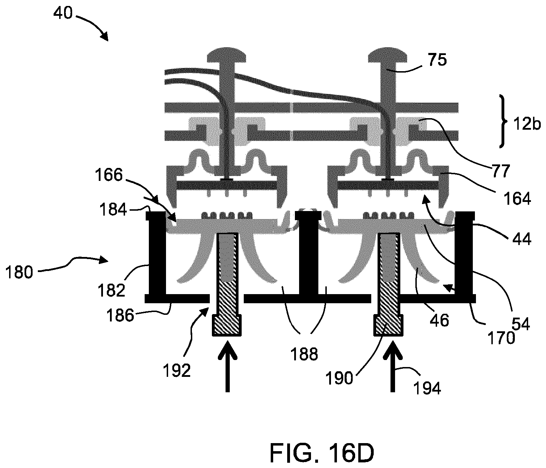

Jig system **180** also comprises a plurality of jig shafts **190** respectively introduced into the plurality of recesses **188** from the side of inner surface **186** via through holes **192** formed in scaffold **182,** in a manner that each jig shaft **190** is able to protrude through a base of the respective recess. In use of jig system **180,** a plurality of disposable electrode assemblies **170** of sensing system **40,** including bodies **54** with legs **46,** are placed in recesses **188,** thereby pushing jig shafts **190** inwardly to protrude out of inner surface **186** of scaffold **182** (FIGs. 16B and 16C). Inner shell **12b** of system **10** is then mounted on scaffold **182** (FIG. 16D). Preferably, the shape and size of scaffold **182,** and the locations of recesses **188** are selected such that once inner shell **12b** is then mounted on the outer surface **184,** each one of housings **164** is aligned with a respective recess **188.** Shafts **190** are then pushed **194** outwardly in the direction of outer surface **184** to release electrode assemblies **170** out of recesses **188** and to connect electrode assemblies **170** to housings **164** by means of snap members **166.**

As used herein the term "about" refers to ± 10 %.

The word "exemplary" is used herein to mean "serving as an example, instance or illustration." Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments." Any particular embodiment of the disclosure may include a plurality of "optional" features unless such features conflict.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular from "a", "an" and "the" include plural references unless the context clearly dictates otherwise. For example, the term "a compound" or "at least one compound" may include a plurality of compounds, including mixtures thereof.

Throughout this application, various embodiments of this disclosure may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

It is appreciated that certain features of the disclosure, which are, for clarity, described in the context of separate embodiments, may also be provided in combination in a single embodiment. Conversely, various features of the disclosure, which are, for brevity, described in the context of a single embodiment, may also be provided separately or in any suitable subcombination or as suitable in any other described embodiment of the disclosure. Certain features described in the context of various embodiments are not to be considered essential features of those embodiments, unless the embodiment is inoperative without those elements.

Various embodiments and aspects of the present disclosure as delineated hereinabove find experimental support in the following examples.

### EXAMPLES

Reference is now made to the following examples, which together with the above descriptions illustrate some embodiments of the disclosure in a non limiting fashion.

These Examples describe stimulation types and the corresponding cortex area at which an ERP signals is induced.

### Example 1

### Electrical stimulation

Table 1, below, lists limb stimulations and the corresponding cortex locations at which an ERP signal can be induced. Following is a representative example for a stimulation protocol that can be used for limb stimulation.

For upper limb median nerve placement a cathode can be placed between the tendons of palmaris longus and flexor carpi radialis, 2 cm proximal to wrist crease, an anode can be placed from about 2 to about 3 cm distal to the cathode or the dorsum of the wrist, and the ground electrode can be metal plate or circumferential band electrode or a stick on electrode placed on the forearm, or to use the ground electrode of the EEG system.

For lower limb posterior tibial nerve placement, a cathode can be placed between the medial border of the Achilles tendon and the posterior border of the medial malleolus, and an anode can be placed 3 cm distal to the cathode.

The following parameters can be used for the electric stimulation: impedance less than 5 KΩ, a ground electrode on the same limb, monophasic rectangular pulses using constant voltage / constant current stimulator, pulse width from about 100µs to about 300 µs, stimulation rate of from about 3 Hz to about 5 Hz, analysis time of about 40 ms for median, and about 60 ms for posterior tibial, number of trials from about 500 to about 1000.

**Table 1**

| Limb | Cortex area |
|---|---|
| Left wrist | hand area in right somatosensory cortex (lateral) |
| Right wrist | hand area in left somatosensory cortex (lateral) |
| Left ankle | leg area in right somatosensory cortex (medial) |
| Right leg | leg area in left somatosensory cortex (medial) |

### Example 2

### Auditory stimulation

Table 2, below, lists auditory stimulations and the corresponding cortex locations at which an ERP signal can be induced. Following is a representative example for a stimulation protocol that can be used for auditory stimulation.

The stimulation can be in the form of audio clicks at the ears of the subject, by conformable earplugs connected to a transducer or in-ear speakers or oscillator arranged to vibrate the bones (bone conduction). Auditory stimulation with broad-band clicks is preferred. Click intensity can be about 100 dB pe SPL or from about 60 to about 70 dB HL. Alternating polarity clicks can be used to reduce artifacts. Stimulus rates of from about 5 clicks per second to about 12 clicks per second. The analysis time can be about 15 ms from stimulus onset. The number of trials from about 500 to about 1000. When stimulating the ears sequentially, the non-stimulated ear is optionally and preferably masked with a white noise at 60 dB pe SPL or from about 30 to about 35 dB HL to eliminate "crossover" responses (*e.g*., bone-conducted responses originating in the non-stimulated ear).

**Table 2**

| Ear | Cortex area |
|---|---|
| Left ear | Left temporal auditory cortex (+pons + midbrain -deep brainstem) |
| Right ear | Right temporal auditory cortex (+pons + midbrain -deep brainstem) |

### Example 3

### Visual stimulation

Table 3, below, lists visual stimulations and the corresponding cortex locations at which an ERP signal can be induced. Following is a representative example for a stimulation protocol that can be used for visual stimulation. Visual stimuli can be used at a rate of about 1 Hz. The stimulus can be presented using a photo-stimulator lamp or a LED matrix, or a screen with a known pattern, optionally and preferably with a stroboscope. Preferably, the sound of the stroboscope is masked by white audio noise.

**Table 3**

| Visual Stimulus | Cortex area |
|---|---|
| Flash | the primary visual cortex (occipital lobe) |
| Pattern | calcarine sulcus next to the primary visual cortex (occipital lobe) |

In addition, citation or identification of any reference in this application shall not be construed as an admission that such reference is available as prior art to the present invention. To the extent that section headings are used, they should not be construed as necessarily limiting.

## Claims

1. A system (10) for measuring electroencephalography (EEG) signals, the system (10) comprising:
a wearable body (12) adapted to fit over a scalp (14) and having an inner shell (12b) and an outer shell (12a);
a plurality of physically separate sensing systems (40) mounted on said wearable body (12), each sensing system (40) having a circuit board (44) and a plurality of flexible legs (46) serving as electrodes for said sensing system (40), wherein said circuit board (44) and said plurality of flexible legs (46) are detachable from each other and are supported by said inner shell (12b);
a plurality of controllable actuators (70) supported by said outer shell (12a) and configured for applying force (42) to said electrodes;
a controller (72) configured for individually controlling each actuator (70) or group of actuators (70) to apply force (42) to at least one electrode; and
a signal processor (18) configured for receiving and processing signals from said electrodes and transmitting control signals to said controller (72) based on said processing.

2. The system (10) of claim 1, wherein said signal processor (18) is configured for determining at least one of: an electrode-tissue impedance, a signal-to-noise ratio, artifacts percentage, and a signal quality, and to control said force (42) based on said determination.

3. The system (10) according to any of claims 1 and 2, wherein said actuator (70) is configured to apply said force (42) while establishing rotary motion to said electrodes.

4. The system (10) according to any of claims 1-3, wherein at least one of said controllable actuators (70) comprises an inflatable balloon, applying said force (42) upon inflation thereof.

5. The system (10) according to any of claims 1-4, wherein said force (42) is periodic and is applied to vibrate said electrodes or generate a hammering effect.

6. The system (10) according to any of claims 1-4, wherein said force (42) is non-periodic.

7. The system (10) according to any of claims 1-6, wherein each flexible leg (46) has a non-conductive section (48) and a conductive section (52) having a tip (50) in electrical communication with said circuit board (44), and wherein each conductive section (52) is one of said electrodes.

8. The system (10) according to claim 7, wherein a conductive section (52) of at least one of said plurality of legs (46) comprises a bundle of conductive bristles.

9. The system (10) according to any of claims 7-8, comprising a controllable vibrating member configured for vibrating said legs (46).

10. The system (10) according to any of claims 7-9, wherein said plurality of flexible legs (46) is arranged on a base of a sensing system body (54), wherein at least one of said sensing systems (40) comprises a shaft (75) and a housing (164) mounted on said shaft (75) and being configured to receive said sensing system body (54), and wherein said housing (164) comprises a rigid wall (165) for holding said sensing system body (54) and a flexible membrane (167) connecting said rigid wall (165) with said shaft (75) in a manner that allows said housing (164) to assume a plurality of different orientations with respect to said shaft (75).

11. The system (10) according to any of claims 1-10, wherein said signal processor (18) is configured to receive from said actuator (70) signals indicative of said force (42).

12. The system (10) according to any of claims 1-11, wherein electrical conductance between said scalp (14) and said circuit board (44) is established after said force (42) is applied, and wherein before said application of said force (42) scalp (14) and circuit board (44) are devoid of electrical conductance therebetween.

13. The system (10) according to any of claims 1-12, wherein said legs are disposable and said circuit board (44) is reusable.

14. The system (10) according to any of claims 1-13, wherein said signal processor (18) is configured to signal said controller (72) to operate said actuator (70) following a user input.

15. A method of measuring electroencephalography (EEG) signals, the method comprising operating the system (10) according to any of claims 1-14, while said wearable body (12) is placed on a scalp (14) of a subject, to receive EEG signals sensed by said plurality of electrodes, thereby measuring the EEG signals.

## Patentansprüche

1. Ein System (10) zur Messung von Elektroenzephalografie (EEG)-Signalen, wobei das System (10) umfasst:
einen tragbaren Körper (12), der so ausgelegt ist, dass er über eine Kopfhaut (14) passt, und der eine Innenhülle (12b) und eine Außenhülle (12a) aufweist;
eine Vielzahl von physisch getrennten Sensorsystemen (40), die an dem tragbaren Körper (12) angebracht sind, wobei jedes Sensorsystem (40) eine Leiterplatte (44) und eine Vielzahl flexibler Schenkel (46) aufweist, die als Elektroden für das jeweilige Sensorsystem (40) dienen, wobei die Leiterplatte (44) und die Vielzahl flexibler Schenkel (46) voneinander abnehmbar sind und von der Innenhülle (12b) gestützt werden;
eine Vielzahl von steuerbaren Aktuatoren (70), die von der genannten Außenhülle (12a) gestützt werden und so ausgelegt, dass sie eine Kraft (42) auf die Elektroden ausüben;
eine Steuerung (72), die so ausgelegt ist, dass sie jeden Aktuator (70) oder jede Gruppe von Aktuatoren (70) einzeln steuert, um eine Kraft (42) auf mindestens eine Elektrode auszuüben; und
einen Signalprozessor (18), der so konfiguriert ist, dass er Signale von den Elektroden empfängt und verarbeitet und auf der Grundlage dieser Verarbeitung Steuersignale an die Steuerung (72) überträgt.

2. System (10) nach Anspruch 1, wobei der Signalprozessor (18) so ausgelegt ist, dass er mindestens einen der folgenden Werte ermittelt: eine Elektroden-Gewebe-Impedanz, ein Signal-Rausch-Verhältnis, einen Artefaktprozentsatz und eine Signalqualität; und die Kraft (42) auf der Grundlage dieser Ermittlung steuert.

3. System (10) nach einem der Ansprüche 1 und 2, wobei der Aktuator (70) so ausgelegt ist, dass er die Kraft (42) ausübt und gleichzeitig eine Drehbewegung der Elektroden bewirkt.

4. System (10) nach einem der Ansprüche 1 bis 3, wobei mindestens einer der steuerbaren Aktuatoren (70) einen aufblasbaren Ballon umfasst, der beim Aufblasen die Kraft (42) ausübt.

5. System (10) nach einem der Ansprüche 1 bis 4, wobei die Kraft (42) periodisch ist und ausgeübt wird, um die Elektroden in Schwingung zu versetzen oder einen Hämmer-Effekt zu erzeugen.

6. System (10) nach einem der Ansprüche 1 bis 4, wobei die Kraft (42) nicht periodisch ist.

7. System (10) nach einem der Ansprüche 1 bis 6, wobei jeder flexible Schenkel (46) einen nichtleitfähigen Abschnitt (48) und einen leitfähigen Abschnitt (52) aufweist mit einer Spitze (50) in elektrischer Verbindung mit der genannten Leiterplatte (44), und wobei jeder leitfähige Abschnitt (52) eine der genannten Elektroden ist.

8. System (10) nach Anspruch 7, wobei ein leitfähiger Abschnitt (52) von mindestens einem der mehreren Schenkel (46) ein Bündel leitfähiger Borsten umfasst.
System (10) nach Anspruch 7, wobei ein leitfähiger Abschnitt (52) mindestens eines der mehreren Schenkel (46) ein Bündel leitfähiger Borsten umfasst.

9. System (10) nach einem der Ansprüche 7 bis 8, umfassend ein steuerbares Schwingungselement, das zum Vibrieren der Schenkel (46) ausgelegt ist.

10. System (10) nach einem der Ansprüche 7 bis 9, wobei die Vielzahl flexibler Schenkel (46) auf einer Basis eines Sensorsystemkörpers (54) angeordnet ist, wobei mindestens eines der Sensorsysteme (40) einen Schaft (75) und ein auf dem Schaft (75) montiertes Gehäuse (164) umfasst, das zur Aufnahme des Sensorsystemkörpers (54) ausgebildet ist, und wobei das Gehäuse (164) eine starre Wand (165) zum Halten des Sensorsystemkörpers (54) und eine flexible Membran (167) umfasst, die die starre Wand (165) mit dem Schaft (75) so verbindet, dass das Gehäuse (164) gegenüber dem Schaft (75) eine Vielzahl unterschiedlicher Ausrichtungen einnehmen kann.

11. System (10) nach einem der Ansprüche 1 bis 10, wobei der Signalprozessor (18) so konfiguriert ist, dass er von dem Aktuator (70) Signale empfängt, die die Kraft (42) anzeigen.

12. System (10) nach einem der Ansprüche 1 bis 11, wobei eine elektrische Leitfähigkeit zwischen der Kopfhaut (14) und der Leiterplatte (44) hergestellt wird, nachdem die Kraft (42) ausgeübt wird, und wobei vor dem Aufbringen der Kraft (42) zwischen der Kopfhaut (14) und der Leiterplatte (44) keine elektrische Leitfähigkeit besteht.

13. System (10) nach einem der Ansprüche 1 bis 12, wobei die Schenkel wegwerfbar sind und die Leiterplatte (44) wiederverwendbar ist.

14. System (10) nach einem der Ansprüche 1 bis 13, wobei der Signalprozessor (18) so konfiguriert ist, dass er der Steuerung (72) signalisiert, den Aktuator (70) nach einer Benutzereingabe zu betätigen.

15. Verfahren zur Messung von Elektroenzephalografie (EEG)-Signale, wobei das Verfahren den Betrieb des Systems (10) nach einem der Ansprüche 1 bis 14 umfasst, während der tragbare Körper (12) auf der Kopfhaut (14) einer Testperson platziert ist, um von der Vielzahl von Elektroden erfassten EEG-Signale zu empfangen und dadurch die EEG-Signale zu messen.

## Revendications

1. Un système (10) destiné à mesurer des signaux d'électroencéphalographie (EEG), le système (10) comprenant :
un corps portable (12) adapté pour s'ajuster sur un cuir chevelu (14) et comportant une coque intérieure (12b) et une coque extérieure (12a) ;
une pluralité de systèmes de détection (40) physiquement séparés, montés sur ledit corps portable (12), chaque système de détection (40) comportant une carte de circuit imprimé (44) et une pluralité de pattes flexibles (46) servant d'électrodes pour ledit système de détection (40), dans lequel ladite carte de circuit imprimé (44) et ladite pluralité de pattes flexibles (46) sont détachables les unes des autres et sont supportées par ladite coque intérieure (12b) ;
une pluralité d'actionneurs contrôlables (70) supportés par ladite coque extérieure (12a) et configurés pour appliquer une force (42) auxdites électrodes ;
un contrôleur (72) configuré pour commander individuellement chaque actionneur (70) ou groupe d'actionneurs (70) afin d'appliquer une force (42) à au moins une électrode ; et
un processeur de signaux (18) configuré pour recevoir et traiter des signaux provenant desdites électrodes et pour transmettre des signaux de commande audit contrôleur (72) sur la base dudit traitement.

2. Système (10) selon la revendication 1, dans lequel ledit processeur de signaux (18) est configuré pour déterminer au moins l'un des éléments suivants : une impédance électrode-tissu, un rapport signal/bruit, un pourcentage d'artefacts et une qualité de signal, et pour commander ladite force (42) sur la base de ladite détermination.

3. Système (10) selon l'une quelconque des revendications 1 et 2, dans lequel ledit actionneur (70) est configuré pour appliquer ladite force (42) tout en établissant un mouvement de rotation auxdites électrodes.

4. Système (10) selon l'une quelconque des revendications 1 à 3, dans lequel au moins l'un desdits actionneurs contrôlables (70) comprend un ballon gonflable, appliquant ladite force (42) lors de son gonflage.

5. Système (10) selon l'une quelconque des revendications 1 à 4, dans lequel ladite force (42) est périodique et est appliquée pour faire vibrer lesdites électrodes ou générer un effet de martèlement.

6. Système (10) selon l'une quelconque des revendications 1 à 4, dans lequel ladite force (42) est non périodique.

7. Système (10) selon l'une quelconque des revendications 1 à 6, dans lequel chaque patte flexible (46) comporte une section non conductrice (48) et une section conductrice (52) dotée d'une pointe (50) en communication électrique avec ladite carte de circuit imprimé (44), et dans lequel chaque section conductrice (52) est l'une desdites électrodes.

8. Système (10) selon la revendication 7, dans lequel une section conductrice (52) d'au moins l'une parmi ladite pluralité de pattes (46) comprend un faisceau de poils conducteurs.

9. Système (10) selon l'une quelconque des revendications 7 à 8, comprenant un élément vibrant commandable configuré pour faire vibrer lesdites pattes (46).

10. Système (10) selon l'une quelconque des revendications 7 à 9, dans lequel ladite pluralité de pattes flexibles (46) est disposée sur une base d'un corps du système de détection (54), dans lequel au moins l'un desdits systèmes de détection (40) comprend un arbre (75) et un boîtier (164) monté sur ledit arbre (75) et conçu pour recevoir ledit corps du système de détection (54), et dans lequel ledit boîtier (164) comprend une paroi rigide (165) destinée à retenir ledit corps du système de détection (54) et une membrane flexible (167) reliant ladite paroi rigide (165) audit arbre (75) de manière à permettre audit boîtier (164) d'adopter une pluralité d'orientations différentes par rapport audit arbre (75).

11. Système (10) selon l'une quelconque des revendications 1 à 10, dans lequel ledit processeur de signaux (18) est configuré pour recevoir dudit actionneur (70) des signaux indicatifs de ladite force (42).

12. Système (10) selon l'une quelconque des revendications 1 à 11, dans lequel une conductance électrique entre ledit cuir chevelu (14) et ladite carte de circuit imprimé (44) est établie après l'application de ladite force (42), et dans lequel, avant ladite application de ladite force (42), le cuir chevelu (14) et la carte de circuit imprimé (44) sont dépourvus de conductance électrique entre eux.

13. Système (10) selon l'une quelconque des revendications 1 à 12, dans lequel lesdites pattes sont jetables et ladite carte de circuit imprimé (44) est réutilisable.

14. Système (10) selon l'une quelconque des revendications 1 à 13, dans lequel ledit processeur de signal (18) est configuré pour envoyer un signal audit contrôleur (72) afin qu'il fasse fonctionner ledit actionneur (70) à la suite d'une entrée de l'utilisateur.

15. Procédé de mesure de signaux d'électroencéphalographie (EEG), le procédé consistant à faire fonctionner le système (10) selon l'une quelconque des revendications 1 à 14, tandis que ledit corps portable (12) est placé sur le cuir chevelu (14) d'un sujet, afin de recevoir les signaux EEG détectés par ladite pluralité d'électrodes, mesurant ainsi les signaux EEG.
